(19) Europäisches Patentamt
European Patent Office
Office européen des brevets

(11) EP 1 621 560 B1

(12) # FASCICULE DE BREVET EUROPEEN

(45) Date de publication et mention de la délivrance du brevet:
**04.06.2008 Bulletin 2008/23**

(51) Int Cl.:
***C08F 220/18*** *(2006.01)*

(21) Numéro de dépôt: **05291493.4**

(22) Date de dépôt: **11.07.2005**

(54) **Copolymère hyperbranché comprenant des monomères sélectionnés, composition le comprenant, et procédé cosmétique**

Hyperverzweigtes polymer, dieses enthaltende Zusammensetzung und kosmetisches Verfahren

Hyperbranched polymer comprising selected monomers, composition and cosmetic process

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **29.07.2004 FR 0408373**

(43) Date de publication de la demande:
**01.02.2006 Bulletin 2006/05**

(73) Titulaire: **L'ORÉAL**
**75008 Paris (FR)**

(72) Inventeur: **Lion, Bertrand**
**75012 Paris (FR)**

(74) Mandataire: **Dodin, Catherine**
**L'OREAL - D.I.P.I.**
**25-29 Quai Aulagnier**
**92600 Asnières (FR)**

(56) Documents cités:
**EP-A- 0 348 565**
**EP-A- 1 428 493**
**WO-A-01/96429**
**WO-A-03/072621**
**WO-A-20/04078809**
**US-A- 4 542 171**

- **HUGENBERG N ET AL: "Synthesis and large scale fractionation of non-linear polymers: brushes and hyperbranched polymers" JOURNAL OF NON-CRYSTALLINE SOLIDS, NORTH-HOLLAND PHYSICS PUBLISHING. AMSTERDAM, NL, vol. 307-310, septembre 2002 (2002-09), pages 765-771, XP004377161 ISSN: 0022-3093**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention a trait à de nouveaux copolymères hyperbranchés, ainsi qu'aux compositions notamment cosmétiques ou pharmaceutiques, en particulier dermatologiques topiques, les comprenant; elle concerne également l'utilisation de ces copolymères notamment dans le domaine du maquillage et/ou du soin des matières kératiniques et plus particulièrement de la peau du corps ou du visage et des lèvres.

Dans le domaine de la cosmétique, on cherche souvent à disposer de compositions permettant d'obtenir un dépôt notamment adhésif ou filmogène, sur les matières kératiniques considérées, telles que la peau, les lèvres, les cheveux, les cils ou les ongles.

En particulier, ces compositions peuvent apporter de la couleur (compositions de maquillage ou de coloration capillaire), de la brillance ou de la matité (compositions de soin ou de maquillage de la peau), des propriétés physiques telles que de la mise en forme (compositions capillaires notamment de coiffage), des propriétés de soin ou de protection (compositions de soin, par exemple d'hydratation ou de protection UV).

On recherche généralement une bonne rémanence et tenue dans le temps du dépôt cosmétique, ainsi qu'une bonne adhésion sur le support. En particulier, il est souhaitable que ce dépôt puisse résister aux agressions mécaniques telles que frottements, transferts par contact d'un autre objet; à l'eau, à la sueur, aux larmes, à la pluie, au sébum et aux huiles. Ceci est particulièrement vrai en maquillage, notamment dans le domaine des rouges à lèvres où l'on recherche la tenue prolongée de la couleur et de la brillance et le non transfert de la couleur; dans le domaine des fonds de teint, fards à paupières et poudres où l'on recherche la tenue de la couleur apportée, en maintenant le plus longtemps possible la matité du maquillage initial malgré la sécrétion de sébum et de sueur, ainsi que le non transfert. En outre, les compositions de maquillage doivent être confortables à porter et ne pas présenter une texture trop collante.

Pour concilier l'ensemble de ces propriétés, souvent antinomiques, au sein d'une même composition, il est possible d'employer un mélange de plusieurs polymères, de nature chimiques très différentes, chaque polymère apportant une des caractéristiques souhaitées. Toutefois, l'emploi d'un mélange de polymères ayant des natures chimiques différentes, pas forcément compatibles, peut générer des problèmes de démixtion au sein de la composition.

L'utilisation de polymères statistiques, par exemple de polymères acryliques conventionnels obtenus par polymérisation radicalaire classique par mélange statistique de monomères, ne permet pas de résoudre ces problèmes de manière satisfaisante. En effet, les polymères statistiques connus dans l'art antérieur présentent une dispersité en composition des chaînes polymériques, ce qui conduit également à une démixtion des polymères au sein de la formule.

On connaît par EP1428493 des compositions cosmétiques comprenant des copolymères à gradient présentant un indice de polydispersité en masse inférieur
ou égal à 2,5 et qui évitent les problèmes de demixtion au sein de la formule tout en apportant les propriétés cosmétiques recherchées.

On connaît également des polymères hyperbranchés qui ont été proposés pour une utilisation en cosmétique capillaire, dans la demande WO01/96429; ces polymères hyperbranchés sont préparés à partir d'un premier type de monomères acryliques et de monomères de branchement possédant deux fonctions polymérisables de réactivités différentes, de façon à obtenir des polymères comportant des motifs allyliques pendants, pouvant être polymérisés ultérieurement, en présence d'un second type de monomères acryliques.

Toutefois, les polymères décrits dans ce document sont solubles dans les milieux aqueux et peuvent difficilement être formulés dans les milieux lipophiles généralement employés en cosmétique, tels que notamment les milieux huileux ou solvants. Or certaines compositions cosmétiques de maquillage, telles que les rouges à lèvres et les fonds de teint comprennent très généralement une phase grasse.

La présente invention a pour but de pallier les inconvénients de l'art antérieur en proposant des nouveaux copolymères hyperbranchés particuliers qui évitent les problèmes de démixtion au sein de la formule tout en permettant d'apporter les propriétés cosmétiques recherchées. Par ailleurs, ces copolymères peuvent aisément être formulés dans les milieux lipophiles des compositions cosmétiques.

En effet, de manière surprenante et inattendue, la Demanderesse a maintenant découvert que certains copolymères hyperbranchés, répondant à des impératifs bien spécifiques en terme de composition permettaient de satisfaire pleinement les exigences indiquées ci-avant.

Un objet de la présente invention est donc un copolymère hyperbranché, comprenant au moins deux, notamment trois, branches polymériques, qui comportent, chacune, au moins un point de ramification au moins trivalent, distinct et indépendant l'un de l'autre, dans lequel :

- une première branche comprend au moins un premier monomère choisi parmi l'acrylate d'isobornyle et le méthacrylate d'isobornyle, et
- au moins une deuxième branche comprend au moins un deuxième monomère, identique ou différent dudit premier monomère, et choisi dans le groupe formé par l'acrylate d'isobornyle, le méthacrylate d'isobomyle, l'acrylate d'isobutyle, le méthacrylate d'isobutyle et l'acrylate d'éthyle-2-hexyle.

Un autre objet de l'invention est une composition cosmétique ou pharmaceutique, notamment dermatologique topique, comprenant, dans un milieu physiologiquement acceptable, notamment cosmétiquement ou dermatologiquement acceptable, au moins un copolymère hyperbranché comprenant au moins deux, notamment trois, branches polymériques, qui comportent, chacune, au moins un point de ramification au moins trivalent, distinct et indépendant l'un de l'autre,

dans lequel une première branche comprend au moins un premier monomère choisi dans le groupe formé par l'acrylate d'isobornyle, le méthacrylate d'isobornyle, l'acrylate d'isobutyle, le méthacrylate d'isobutyle et l'acrylate d'éthyle-2-hexyle, et au moins une deuxième branche comprend au moins un deuxième monomère, identique ou différent dudit premier monomère, et choisi dans ledit groupe.

Les polymères selon l'invention présentent l'avantage de permettre l'obtention d'un film particulièrement souple, et qui n'est pas du tout collant.

Les "polymères hyperbranchés" au sens de l'invention peuvent être définis comme des polymères comprenant au moins deux, notamment trois, branches polymériques, formant indifféremment la branche principale ou une branche secondaire, et comportant, chacune, au moins un point de ramification au moins trivalent, distinct et indépendant, et qui peut former au moins deux points de ramification au moins trivalents, distincts et indépendants l'un de l'autre. Chaque point de ramification est de préférence disposé au coeur d'au moins une chaîne.

Les branches sont de préférence reliées entre elles par un composé multifonctionnel, notamment tel que défini plus loin dans la description, et qui présente de préférence des réactivités différentes.

Au sens de la présente invention, on entend par "point de ramification trivalent", le point de jonction entre trois branches polymériques, dont au moins deux branches sont de constitution chimique et/ou de structure différentes. Par exemple, certaines branches peuvent être hydrophiles, c'est-à-dire comporter en majorité des monomères hydrophiles et d'autres branches peuvent être hydrophobes, c'est-à-dire comporter en majorité des monomères hydrophobes. D'autres branches encore peuvent former un polymère statistique ou un polymère bloc.

Par analogie, on entend par point de "ramification au moins trivalent", les points de jonction entre au moins trois branches polymériques, par exemple n branches polymériques, dont n-1 branches au moins sont de constitution chimique et/ou de structure différentes.

On entend par "coeur de chaîne", les atomes situés à l'intérieur de la chaîne polymérique, à l'exclusion des atomes formant les deux extrémités de cette chaîne.

On entend par "branche principale", la branche ou séquence polymérique comprenant le % en poids de monomère(s) le plus important.

Les branches qui ne sont pas principales sont nommées "branches secondaires".

La définition conforme à l'invention de la notion de "polymère hyperbranché" n'englobe pas, notamment:

- les polymères branchés ou à greffons, c'est à dire les polymères constitués d'une chaîne principale présentant de multiples points de ramifications trivalents dont chacun est le point de départ d'une chaîne latérale linéaire, les chaînes latérales pouvant être constituées de un ou plusieurs blocs, ces chaînes latérales étant de nature identique ou différente de la chaîne principale, comme par exemple ceux décrits dans le document EP815848, relatifs à des copolymères à squelette carboné/fluoré de Tg compris entre 0 et 30°C et à greffons rigides de Tg supérieure à 25°C ou dans le document WO 97/35541, relatif à des copolymères à squelette vinylique/acrylique rigide, hydrophile et à greffons flexibles hydrophobes;
- les polymères en peigne, (cas particulier des polymères à greffons) c'est à dire les polymères constitués d'une chaîne principale présentant de multiples points de ramifications trivalents dont chacun est le point de départ d'une chaîne latérale linéaire (Glossary of basic terms in polymer science/IUPAC/1996), les points de ramifications étant situés à intervalles réguliers;
- les polymères étoiles, c'est-à-dire ceux dont tous les points de ramifications se trouvent localisés en un seul point.

Dans le cadre de la présente invention, on préfère les polymères hyperbranchés comprenant au moins deux branches polymériques, formant indifféremment la branche principale ou une branche secondaire, et comportant, chacune, au moins un point de ramification trivalent, distinct et indépendant l'un de l'autre, qui peut former au moins deux points de ramification trivalents, distincts et indépendants l'un de l'autre; chaque point de ramification étant disposé de préférence au coeur d'au moins une chaîne.

Sans être tenu par la présente illustration, on peut considérer que les copolymères selon l'invention peuvent être illustrés par le schéma suivant :

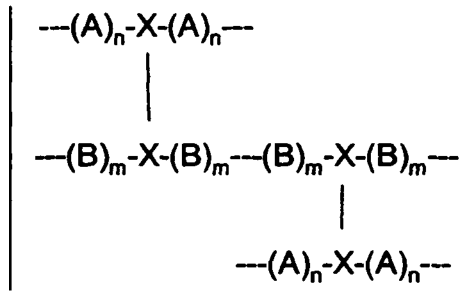

dans lequel A et B représentent les monomères, sélectionnés et/ou additionnels tels que ci-après définis, m et n étant leur degré de polymérisation, et X représente le point de ramification trivalent, présent sur chacune des branches, et pouvant découler d'un composé multifonctionnel tel que défini ci-après.

Les copolymères selon invention présentent l'avantage, au moins pour une partie d'entre eux, de pouvoir facilement être mis en oeuvre dans les milieux cosmétiques organiques, notamment comprenant des solvants lipophiles et/ou des huiles cosmétique, tout en conservant des propriétés rhéologiques intéressantes.

En particulier, les copolymères selon l'invention présentent une bonne solubilité dans les solvants en particulier lipophiles et/ou dans les huiles cosmétiques.

On rappelle que par "polymère soluble" dans un milieu, on entend que le polymère ne forme pas de précipité, mais forme avantageusement une solution limpide, dans ledit milieu, à 25°C.

Avantageusement, le copolymère selon l'invention est soluble à une concentration d'au moins 3% en poids, dans l'isododécane, à 25°C, 1 atm., de préférence à une concentration d'au moins 5% en poids, voire d'au moins 10% en poids.

Les copolymères selon l'invention sont donc des copolymères hyperbranchés c'est-à-dire comprenant au moins deux, notamment au moins trois, branches polymériques, qui peuvent former indifféremment la branche principale ou une branche secondaire, et comportant, chacune, au moins un point de ramification au moins trivalent, distinct et indépendant l'un de l'autre, notamment pour former au moins deux points de ramification au moins trivalents, distincts et indépendants l'un de l'autre.

Avantageusement, les copolymères hyperbranchés selon l'invention comprennent des motifs dérivés d'un ou de plusieurs monomères éthyléniques capables en particulier de polymériser par voie radicalaire pour former les branches dudit polymère, chaque branche pouvant se présenter sous la forme d'une séquence polymérique de nature homopolymère, ou copolymère statistique, alterné, bloc

ou gradient; de préférence, chaque branche se présente sous la forme d'un homopolymère ou d'un copolymère statistique linéaire.

Les copolymères selon l'invention comprennent au moins une première branche

ou séquence polymérique comprenant au moins un premier monomère sélectionné, et au moins une deuxième séquence (ou deuxième branche) comprenant au moins un deuxième monomère sélectionné, qui peut être identique ou différent dudit premier monomère sélectionné.

Par "monomère sélectionné", on entend dans la présente invention, les monomères choisis dans la liste suivante : l'acrylate d'isobornyle, le méthacrylate d'isobornyle, l'acrylate d'isobutyle, le méthacrylate d'isobutyle et l'acrylate d'éthyle-2-hexyle.

Chaque branche ou séquence peut bien évidemment comprendre un mélange de monomères sélectionnés, chaque branche pouvant avoir une composition chimique et/ou une structure distincte.

On préfère tout particulièrement les copolymères dans lesquels la branche principale se présente sous la forme d'un homopolymère ou d'un copolymère statistique comprenant 80-100% en poids, de préférence 100% en poids, par rapport au poids de la branche, de monomère(s) sélectionnés.

On préfère également les copolymères dans lesquels la ou les branche(s) secondaire(s) se présentent sous la forme d'un homopolymère ou d'un copolymère statistique comprenant 80-100% en poids, de préférence 100% en poids, par rapport au poids de la branche, de monomère(s) sélectionnés.

Le copolymère selon l'invention peut donc comprendre une première branche qui va comprendre un premier monomère sélectionné, ou bien un mélange d'au moins deux monomères sélectionnés, voire de trois monomères sélectionnés,

ou encore un mélange d'un ou deux monomères sélectionnés avec un ou plusieurs monomères additionnels tels que définis ci-après.

Le copolymère selon l'invention peut comprendre également au moins une seconde branche qui peut comprendre un monomère sélectionné distinct dudit premier monomère sélectionné ou identique audit premier monomère sélectionné, qui peut également être seul ou en mélange avec un ou plusieurs autres monomères sélectionnés, et/ou avec un ou

plusieurs monomères additionnels tels que définis ci-après.

D'une manière générale, la quantité totale de monomères choisis parmi les monomères sélectionnés dans le copolymère final est de préférence comprise entre 50 et 100% en poids, notamment de 60 à 98% en poids, voire de 70 à 97% en poids, encore mieux de 80 à 96% en poids, préférentiellement de 90 à 95% en poids, de monomères sélectionnés par rapport au poids total de monomères présents dans le copolymère final.

Le copolymère selon l'invention peut notamment comprendre 25% à 75% en poids, par exemple 30% à 70% en poids d'un premier monomère sélectionné, présent dans une première branche, et 25% à 75% en poids, notamment 30% à 70% en poids d'un second monomère sélectionné, qui peut être présent dans la même branche ou dans une autre branche; les % étant donnés par rapport au poids total de monomères présents dans le copolymère final.
Il peut en outre comprendre, de manière optionnelle, de 1 à 40% en poids, notamment de 5 à 30% en poids, voire de 10 à 25% en poids, par rapport au poids du copolymère final, d'un troisième monomère choisi parmi les monomères sélectionnés; les % étant donnés par rapport au poids total de monomères présents dans le copolymère final.

On préfère tout particulièrement les copolymères comprenant, que cela soit dans la même branche ou dans des branches différentes, les associations de monomères suivants :

- de l'acrylate d'isobornyle et du méthacrylate d'isobornyle;
- de l'acrylate d'isobomyle et de l'acrylate d'isobutyle;
- de l'acrylate d'isobornyle et du méthacrylate d'isobutyle;
- de l'acrylate d'isobornyle et de l'acrylate d'éthyle-2-hexyle;
- du méthacrylate d'isobornyle et de l'acrylate d'isobutyle;
- du méthacrylate d'isobomyle et du méthacrylate d'isobutyle;
- du méthacrylate d'isobornyle et de l'acrylate d'éthyle-2-hexyle;
- de l'acrylate d'isobomyle, du méthacrylate d'isobornyle et de l'acrylate d'éthyle-2-hexyle;
- de l'acrylate d'isobornyle, du méthacrylate d'isobomyle et de l'acrylate d'isobutyle;
- de acrylate d'isobornyle, du méthacrylate d'isobutyle et de l'acrylate d'éthyle-2-hexyle;
- de l'acrylate d'isobomyle, du méthacrylate d'isobutyle et de l'acrylate d'isobutyle; et
- du méthacrylate d'isobornyle, du méthacrylate d'isobutyle et de l'acrylate d'isobutyle.

Très préférentiellement, on peut citer les copolymères comprenant :

- de l'acrylate d'isobornyle dans les branches principales et de l'acrylate d'isobutyle dans les branches secondaires;
- de l'acrylate d'isobornyle dans les branches principales et de l'acrylate d'éthyle-2-hexyle dans les branches secondaires;
- du méthacrylate d'isobornyle dans les branches principales et de l'acrylate d'isobutyle dans les branches secondaires;
- du méthacrylate d'isobornyle dans les branches principales et de l'acrylate d'éthyle-2-hexyle dans les branches secondaires;
- de acrylate d'isobomyle et du méthacrylate d'isobomyle dans les branches principales et de l'acrylate d'isobutyle dans les branches secondaires;
- de l'acrylate d'isobomyle et du méthacrylate d'isobomyle dans les branches principales et de l'acrylate d'éthyle-2-hexyle dans les branches secondaires;
- de l'acrylate d'isobomyle et du méthacrylate d'isobutyle dans les branches principales et de l'acrylate d'isobutyle dans les branches secondaires;
- de l'acrylate d'isobornyle et du méthacrylate d'isobutyle dans les branches principales et de l'acrylate d'éthyle-2-hexyle dans les branches secondaires;
- du méthacrylate d'isobomyle et du méthacrylate d'isobutyle dans les branches principales et de l'acrylate d'isobutyle dans les branches secondaires; ou
- du méthacrylate d'isobornyle et du méthacrylate d'isobutyle dans les branches principales et de l'acrylate d'éthyle-2-hexyle dans les branches secondaires.

Le copolymère selon l'invention peut en outre comprendre au moins un monomère additionnel autre que ceux choisis parmi les monomères sélectionnés.
Ce monomère additionnel, ou mélange de monomères additionnels, peut être présent en une quantité de 0 à 50% en poids, notamment de 2 à 40% en poids, voire de 3 à 30% en poids, encore mieux de 4 à 20% en poids, préférentiellement de 5 à 10% en poids, par rapport au poids total de monomères présents dans le copolymère final.

Ce ou ces monomères additionnels peuvent être choisis parmi, seul ou en mélange, les monomères suivants, ainsi que leurs sels, à l'exclusion bien sûr des monomères sélectionnés ci-dessus mentionnés :

- (i) les (méth)acrylates de formule $CH_2$=CHCOOR ou $CH_2$=C($CH_3$)COOR dans laquelle R représente :

  - un groupe alkyle linéaire ou ramifié, comprenant 1 à 30 atomes de carbone dans lequel se trouve(nt) éventuellement intercalé(s) un ou plusieurs hétéroatomes choisis parmi O, N, S et P et/ou ledit groupe alkyle pouvant être éventuellement substitué par un ou plusieurs substituants choisis parmi -OH, les atomes d'halogène (Cl, Br, I et F), les groupes -NR4R5, où R4 et R5 identiques ou différents représentent l'hydrogène ou un groupe alkyle linéaire ou ramifié, en C1 à C6 ou un groupe phényle; et/ou les groupes polyoxyalkylènes, notamment polyoxyéthylène et/ou polyoxypropylène, ledit groupe polyoxyalkylène étant constitué par la répétition de 5 à 30 motifs oxyalkylène;
  - un groupe cycloalkyle en C3 à C12, ledit groupe cycloalkyle pouvant comporter dans sa chaîne un ou plusieurs hétéroatomes choisis parmi O, N, S et/ou P, et/ou pouvant être éventuellement substitué par un ou plusieurs substituants choisis parmi -OH et les atomes d'halogène (Cl, Br, I et F);
  - un groupe aryle en C4 à C20 ou aralkyle en C5 à C30 (groupe alkyle en C1 à C8);

  notamment R peut être un groupe méthyle, éthyle, propyle, isopropyle, n-butyle, isobutyle, tertiobutyle, hexyle, éthylhexyle, octyle, lauryle, isooctyle, isodécyle, dodécyle, cyclohexyle, t-butylcyclohexyle, stéaryle, éthyl-2-perfluorohexyle, 2-hydroxyéthyle, 2-hydroxybutyle, 2-hydroxypropyle, méthoxyéthyle, éthoxyéthyle, méthoxypropyle, isobornyle, phényle, 2-phényl-éthyle, t-butylbenzyle, benzyle, furfurylméthyle ou tétrahydrofurfurylméthyle, méthoxypolyoxyéthylène (ou POE-méthyle); POE-béhényle, trifluoroéthyle; diméthylaminoéthyle, diéthylaminoéthyle, diméthylaminopropyle.

- (ii) les (méth)acrylamides de formule $CH_2$=CHCON$R_4R_5$ ou $CH_2$=C($CH_3$)CON$R_4R_5$
  dans laquelle R4 et R5, identiques ou différents, représentent un atome d'hydrogène ou :

  a) un groupe alkyle, linéaire ou ramifié, comportant de 1 à 18 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (Cl, Br, I et F), et les groupes Si (R4R5), où R4 et R5 identiques ou différents représentent un groupe alkyle en C1 à C6 ou un groupe phényle ;
  et notamment un groupe méthyle, éthyle, propyle, isopropyle, n-butyle, isobutyle, tertiobutyle, hexyle, isohexyle, cyclohexyle, éthylhexyle, octyle, isooctyle, décyle, isodécyle, cyclodécyle, dodécyle, cyclododécyle, isononyle, lauryle, t-butylcyclohexyle, stéaryle; éthyl-2-perfluorohexyle; ou un groupe hydroxyalkyle en C1-4 tel que 2-hydroxyéthyle, 2-hydroxybutyle et 2-hydroxypropyle; ou un groupe alcoxy(Cl-4)-alkyle(Cl-4) tel que méthoxyéthyle, éthoxyéthyle et méthoxy-propyle,
  b) un groupe cycloalkyle en C3 à C12, tel que le groupe isobornyle, ou un groupe hétérocyctoalkyle (alkyle de 1 à 4 atomes de carbone), tel que furfurylméthyle ou tétrahydrofurfurylméthyle,
  c) un groupe aryle en C4 à C20 tel que le groupe phényle,
  d) un groupe aralkyle en C5 à C30 (groupe alkyle en C1 à C8) tel que 2-phényl éthyle, t-butylbenzyle ou benzyle,

- (iii) les monomères à insaturation(s) éthylénique(s) comprenant au moins une fonction acide carboxylique, phosphorique ou sulfonique, ou anhydride, comme par exemple l'acide acrylique, l'acide méthacrylique, l'acide crotonique, l'anhydride maléique, l'acide itaconique, l'acide fumarique, l'acide maléique, l'acide styrènesulfonique, l'acide vinylbenzoïque, l'acide vinylphosphorique, l'acide acrylamidopropanesulfonique; et les sels de ceux ci;

- (iv) les éthers de vinyle de formule $R_6$O-CH=$CH_2$ ou les esters de vinyle de formule : $R_6$-COO-CH=$CH_2$
  dans laquelle $R_6$ représente un groupe alkyle linéaire ou ramifié, comprenant de 1 à 22 atomes, ou un groupe alkyle cyclique comportant de 3 à 6 atomes de carbone et/ou un groupe aromatique, par exemple de type benzénique, anthracénique, et naphtalénique ;

- (v) les composés vinyliques de formules : CH2=CH-R9, CH2=CH-CH2-R9 ou CH2=C(CH3)-CH2-R9
  dans lesquelles R9 est un groupe hydroxyle, halogène (Cl ou F), NH2, OR10 où R10 représente un groupe phényle ou un groupe alkyle en C1 à C12 (le monomère est un éther de vinyle ou d'allyle); acétamide (NHCOCH3); un groupe OCOR11 où R11 représente un groupe alkyle de 2 à 12 carbones, linéaire ou ramifié (le monomère est un ester de vinyle ou d'allyle); ou un groupe choisi parmi :

  - un groupe alkyle linéaire ou ramifié, de 1 à 18 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes

d'halogène (Cl, Br, I et F), et les groupes Si (R4R5), où R4 et R5 identiques ou différents représentent un groupe alkyle en C1 à C6 ou un groupe phényle ;

- un groupe cycloalkyle en C3 à C12 tel que isobornyle, cyclohexane,
- un groupe aryle en C3 à C20 tel que phényle,
- un groupe aralkyle en C4 à C30 (groupe alkyle en C1 à C8) tel que 2-phényléthyle ; benzyle,
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle de 1 à 4 C), tel que furfurylméthyle ou tétrahydrofurfurylméthyle,

- (vi) le styrène et ses dérivés, notamment tels que le méthylstyrène, le chlorostyrène ou le chlorométhylstyrène;

- (vii) les monomères à insaturation éthylénique comprenant un ou plusieurs atomes de silicium tels que le méthacryloxypropyl triméthoxy silane, le méthacryloxypropyl tris(triméthylsiloxy)silane;

- ainsi que leurs sels et leurs mélanges.

Parmi ces monomères additionnels, on peut tout particulièrement citer les (méth)acrylates de méthyle, d'éthyle, de n-propyle, d'isopropyle, de n-butyle, de t-butyle, de cyclohexyle, de méthoxyéthyle, d'éthoxyéthyle, de trifluoroéthyle, de diméthylaminoéthyle, de diéthylaminoéthyle, de 2-hydroxypropyle, de 2-hydroxyéthyle; l'acide acrylique, l'acide méthacrylique, le (méth)acrylamide, le méthacryloxypropyl triméthoxy silane, le méthacryloxypropyl tris( triméthylsiloxy) silane; ainsi que leurs sels; et leurs mélanges.

Comme monomères additionnels, on peut également employer des macromonomères carbonés ou siliconés ayant au moins un groupe terminal polymérisable. Il s'agit de tout polymère, notamment oligomère, comportant sur une seule de ses extrémités un groupe terminal, notamment polymérisable, apte à réagir lors de la réaction de polymérisation avec les monomères considérés, pour former les chaînes latérales du polymère; ledit groupe terminal peut être avantageusement un groupe à insaturation éthylénique susceptible de se polymériser par voie radicalaire avec les monomères constituant le squelette. Ledit macromonomère permet de former les chaînes latérales du copolymère. Le groupe polymérisable du macromonomère peut être avantageusement un groupe à insaturation éthylénique susceptible de se polymériser par voie radicalaire. Ledit groupe terminal polymérisable peut être en particulier un groupe vinyle ou (méth) acrylate. Parmi les macromonomères additionnels susceptibles d'être employés, on peut notamment citer, seuls ou en mélange, ainsi que leurs sels, :

-(i) les homopolymères et les copolymères (méth)acrylate d'alkyle linéaire ou ramifié en C8-C22, présentant un groupe terminal polymérisable choisi parmi les groupes vinyle ou (méth)acrylate parmi lesquels on peut citer les macromonomères de poly(acrylate d'éthyl-2 hexyle) à extrémité mono(méth)acrylate ; les macromonomères de poly(acrylate de dodécyle) ou de poly(méthacrylate de dodécyle) à extrémité mono(méth)acrylate; les macromonomères de poly(acrylate de stéaryle) ou de poly (méthacrylate de stéaryle) à extrémité mono(méth)acrylate. De tels macromonomères sont notamment décrits dans les brevets EP895467 et EP96459.

-(ii) les polyoléfines ayant un groupe terminal à insaturation éthylénique, en particulier ceux ayant un groupement terminal (méth)acrylate. Comme exemple de telles polyoléfines, on peut citer en particulier les macromonomères suivants, étant entendu qu'ils ont un groupe terminal (méth)acrylate: les macromonomères de polyéthylène, les macromonomères de polypropylène, les macromonomères de copolymère polyéthylène/polypropylène, les macromonomères de copolymère polyéthylène/polybutylène, les macromonomères de polyisobutylène; les macromonomères de polybutadiène; les macromonomères de polyisoprène; les macromonomères de polybutadiène; les macromonomères de poly(éthylène/butylène)-polyisoprène. De tels macromonomères sont en particulier décrits dans US 5,625,005 qui mentionne des macromonomères éthylène/butylène et éthylène/propylène à groupement terminal réactif (méth)acrylate. On peut en particulier citer le méthacrylate de poly(éthylène/butylène), tel que celui commercialisé sous la dénomination Kraton Liquid L-1253 par Kraton Polymers.

-(iii) les polydiméthylsiloxanes à groupement terminal mono(méth)acrylate, et notamment ceux de formule (IIa) suivante :

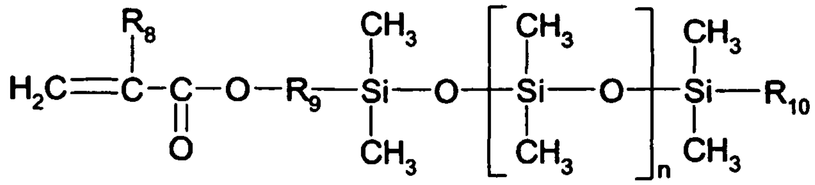

(IIa)

dans laquelle :

- $R_8$ désigne un atome d'hydrogène ou un groupement méthyle; de préférence méthyle;
- $R_9$ désigne un groupe hydrocarboné divalent, linéaire ou ramifié, de préférence linéaire, ayant de 1 à 10 atomes de carbone et contenant éventuellement une ou deux liaisons éther -O- ; de préférence éthylène, propylène ou butylène;
- $R_{10}$ désigne un groupe alkyle linéaire ou ramifié, ayant de 1 à 10 atomes de carbone, notamment de 2 à 8 atomes de carbone; de préférence méthyle, éthyle, propyle, butyle ou pentyle;
- n désigne un nombre entier allant de 1 à 300, de préférence allant de 3 à 200, et préférentiellement allant de 5 à 100.

Comme macromonomères siliconés, on peut en particulier citer les monométhacryloyloxypropyl polydiméthylsiloxanes tels que ceux commercialisés sous la dénomination PS560-K6 par UCT (United Chemical Technologies Inc.) ou sous la dénomination MCR-M17 par Gelest Inc.

Parmi les sels, on peut citer ceux obtenus par neutralisation des groupements acides à l'aide de base minérales telles que LiOH, NaOH, KOH, Ca(OH)2, NH40H ou Zn(OH)2; ou par une base organique telle qu'une alkylamine primaire, secondaire ou tertiaire, notamment la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine et la diméthylamino-2-propanol. On peut encore citer la lysine ou la 3-(diméthylamino)-propylamine.

On peut également peut citer les sels d'acides minéraux, tels que l'acide sulfurique, l'acide chlorhydrique, l'acide bromhydrique, l'acide iodhydrique, l'acide phosphorique, l'acide borique. On peut aussi citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

De préférence, le copolymère selon l'invention comprend au moins une séquence polymérique ou branche dite "de Tg haute", c'est-à-dire ayant une Tg supérieure ou égale à 20°C, notamment comprise entre 20°C et 150°C inclus, notamment comprise entre 30 et 120°C, plus particulièrement comprise entre 40°C et 100°C.

Cette branche est de préférence présente en une quantité supérieure ou égale à 50%, notamment de 55% à 95%, préférentiellement de 60% à 90% en poids, par rapport au poids total du polymère.

De préférence également, le copolymère selon l'invention comprend au moins une séquence ou branche dite "de Tg basse", c'est-à-dire ayant une Tg strictement inférieure à 20°C, c'est-à-dire comprise entre -150°C et 19°C, notamment comprise entre -100°C et 0°C, plus particulièrement comprise entre -80°C et - 5°C, voire comprise entre -70°C et -10°C.

Cette branche est de préférence présente en une quantité inférieure à 50%, notamment de 5% à 45%, préférentiellement de 10% à 40% en poids, par rapport au poids total du polymère.

De préférence, la branche de "Tg haute" comprend essentiellement des monomères dits "de Tg haute" tels que définis ci-dessous, qui peuvent être présents à raison de 30% à 100% en poids, notamment 40 à 95% en poids, encore mieux de 50 à 90% en poids, par rapport au poids total de ladite branche.

De façon similaire, la branche de "Tg basse" comprend essentiellement des monomères dits "de Tg basse" tels que définis ci-dessous, et qui peuvent être présents à raison de 30% à 100% en poids, notamment 40 à 95% en poids, encore mieux de 50 à 90% en poids, par rapport au poids total de ladite branche.

Il est à noter qu'on préfère tout particulièrement les copolymères dans lesquels la branche principale est "de Tg haute" et au moins une des branches secondaires est "de Tg basse".

Par ailleurs, les copolymères selon l'invention comprennent de préférence au moins un monomère dit "de Tg haute", c'est-à-dire ayant une Tg supérieure ou égale à 20°C, notamment comprise entre 20 et 150°C, plus particulièrement comprise entre 30 et 120°C, et encore mieux comprise entre 40 et 100°C, ou un mélange de tels monomères. Ce ou ces monomères dits "de Tg haute" peuvent être choisis parmi les monomères sélectionnés et/ou parmi les

monomères additionnels.

Le ou les monomères dits "de Tg haute" peuvent être présents à raison de 40 à 100% en poids, notamment 50 à 80% en poids, encore mieux 55 à 70% en poids, par rapport au poids total de monomères présents dans le copolymère.

Les copolymères selon l'invention peuvent donc également comprendre au moins un monomère dit "de Tg basse", c'est-à-dire ayant une Tg strictement inférieure à 20°C, notamment comprise entre -150 et 19°C, plus particulièrement comprise entre -100 et 0°C, encore mieux comprise entre -80 et -5°C, voire comprise entre -70°C et -10°C, ou un mélange de tels monomères, et qui peu(ven)t donc être présent(s) à raison de 0 à 60% en poids, notamment de 20 à 50% en poids, encore mieux de 30 à 45% en poids, par rapport au poids total de monomères présents dans le copolymère.

Ces monomères dits "de Tg basse" peuvent être choisis parmi les monomères sélectionnés et/ou parmi les monomères additionnels.

A titre d'information, on notera que l'acrylate d'isobomyle a une Tg de 94°C, le méthacrylate d'isobornyle a une Tg de 110°C, et le méthacrylate d'isobutyle a une Tg de 53°C, et sont donc dits de Tg haute, supérieure à 20°C, alors que l'acrylate d'isobutyle a une Tg de -24°C et l'acrylate d'éthyle-2-hexyle a une Tg de -50°C et sont donc dits de Tg basse, inférieure à 20°C.

Dans la présente description, on désigne par "monomère de Tg" les monomères dont l'homopolymère a une telle Tg. Dans la présente invention, les Tg (ou température de transition vitreuse) sont des Tg théoriques déterminées à partir des Tg théoriques des monomères constitutifs de chacune des séquences, que l'on peut trouver dans un manuel de référence tel que le Polymer Handbook, 3rd ed, 1989, John Wiley, selon la relation suivante, dite Loi de Fox :

$$\frac{1}{Tg} = \sum_i \left(\frac{\varpi i}{Tgi}\right)$$

wi étant la fraction massique du monomère i dans la séquence considérée et Tgi étant la température de transition vitreuse de l'homopolymère du monomère i (en kelvin).

Préférentiellement, le copolymère selon l'invention comprend un ou plusieurs monomères lipophiles, qui peuvent être choisis parmi les monomères sélectionnés et/ou parmi les monomères additionnels et qui peuvent être présents à raison de 40 à 100% en poids, notamment 50 à 80% en poids, encore mieux 55 à 70% en poids, par rapport au poids total de monomères présents dans le copolymère.

Le copolymère selon l'invention peut donc comprendre un ou plusieurs monomères hydrophiles, qui peuvent être choisis parmi les monomères sélectionnés et/ou parmi les monomères additionnels et qui peuvent être présents à raison de 0 à 60% en poids, notamment 20 à 50% en poids, encore mieux 30 à 45% en poids, par rapport au poids total de monomères présents dans le copolymère.

De préférence, le copolymère selon l'invention comprend 100% en poids de monomères lipophiles, qui peuvent être choisis parmi les monomères sélectionnés et/ou parmi les monomères additionnels.

Il est à noter qu'on préfère tout particulièrement les copolymères dans lesquels la branche principale est lipophile, et encore mieux ceux pour lesquels l'ensemble des branches, principale et secondaires, sont lipophiles.

A titre d'information, on notera que l'acrylate d'isobomyle, le méthacrylate d'isobomyle, le méthacrylate d'isobutyle, l'acrylate d'isobutyle, et l'acrylate d'éthyle-2-hexyle sont tout particulièrement lipophiles.

Dans la présente invention, on entend par monomère lipophile, tout monomère non hydrophile.

Par monomère hydrophile, on entend tout monomère dont l'homopolymère est hydrosoluble c'est-à-dire ne forme pas de précipité, mais forme avantageusement une solution limpide, dans l'eau, à 25°C, 1 atm., à une concentration d'au moins 1 % en poids.

L'homme du métier saura choisir les monomères et leurs quantités en fonction du résultat recherché, en se basant sur ses connaissances générales, notamment sur la réactivité relative de chaque monomère, sur sa Tg et sur sa lipophilie. Tout particulièrement, il choisira les monomères et leur quantité, ainsi que le milieu solvant, de manière à obtenir un copolymère soluble dans ledit milieu solvant.

On notera toutefois que, lorsque le copolymère selon l'invention comprend de l'acide acrylique et/ou de l'acide méthacrylique, ce(s) monomère(s) est/sont de préférence présent(s) en une quantité maximum de 20% en poids, notamment une quantité inférieure à 15% en poids, voire inférieure à 10% en poids, par rapport au poids total de monomères présents dans le copolymère, de manière à conserver sa lipophilie au copolymère final.

De préférence, la masse moléculaire moyenne en nombre du copolymère selon invention est comprise entre 2000 g/mol et 1 500 000 g/mol, notamment entre 5500 g/mol et 1 000 000 g/mol, et encore mieux entre 6000 g/mol et 900 000 g/mol.

On détermine la masse moléculaire moyenne en poids (Mw) et nombre (Mn) par chromatographie liquide par perméation de gel (GPC), éluant THF, courbe d'étalonnage établie avec des étalons de polystyrène linéaire, détecteur réfractométrique.

Les copolymères hyperbranchés selon l'invention peuvent être obtenus à partir de procédés de polymérisation en eux-mêmes connus de l'homme de l'art.

On peut tout particulièrement citer un procédé comprenant au moins une étape consistant en une polymérisation radicalaire en présence de composé multifonctionnel.
On peut ainsi faire réagir:

- le ou les monomères éthyléniques envisagés pour former le copolymère, ou de préférence une partie d'entre eux, à savoir le ou les monomères sélectionnés et/ou additionnels tels que définis ci-dessus; et
- au moins un composé multifonctionnel, c'est-à-dire comportant au moins deux groupes fonctionnels polymérisables ou insaturations, composé aussi appelé "chain extender" et défini ci-après.

Il est possible d'effectuer la réaction en deux étapes, la première étape consistant à copolymériser les monomères envisagés pour former la branche principale, en présence d'une faible quantité de composé multifonctionnel; de préférence, la réactivité des groupes fonctionnels dudit composé multifonctionnel est telle que les monomères envisagés pour former la branche principale vont préférentiellement réagir avec un premier groupe fonctionnel, laissant au moins un des autres groupes fonctionnels non réagi.
Dans une seconde étape, on peut ajouter les monomères susceptibles de former les autres branches, qui vont préférentiellement réagir avec le groupe fonctionnel non réagi du composé multifonctionnel, de manière à former le copolymère hyperbranché. Il est possible dans cette seconde étape, d'ajouter à nouveau du composé multifonctionnel.

De façon préférentielle, les fonctions du composé multifonctionnel présentent des réactivités différentes. Par exemple, le composé multifonctionnel peut comporter une fonction (méth)acrylate qui va polymériser rapidement, et une fonction allylique qui va polymériser plus lentement.
Comme exemple de composé multifonctionnel, on peut en particulier citer les composés de formule suivante (I):

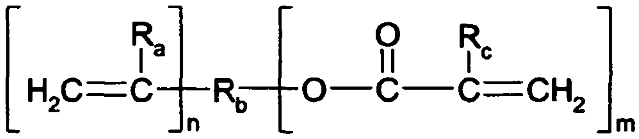

(I)

dans laquelle :

- Ra et Rc sont, indépendamment l'un de l'autre choisi parmi l'hydrogène ou les radicaux alkyles, linéaires ou ramifiés, ayant 1 à 22 atomes de carbone, notamment 1 à 3 atomes de carbone; de préférence Ra peut être un atome d'hydrogène; de préférence Rc peut être un radical méthyle;
- Rb est un groupe divalent alkylène linéaire ou ramifié, ayant 1 à 22 atomes de carbone, ou cycloalkylène ayant 3 à 6 atomes de carbone, ou arylène ayant 6 à 18 atomes de carbone, ou alkarylène ayant 7 à 24 atomes de carbone; de préférence Rb est un groupe méthylène;
- m et n sont, indépendamment l'un de l'autre, égaux à 1, 2, 3 ou 4, et tels que la somme m+n est supérieure ou égale à 2; de préférence m=n=1.

Comme composé multifonctionnel ou "chain extender", on préfère tout particulièrement les (méth)acrylate d'allyle, et encore mieux le méthacrylate d'allyle.

Ledit composé multifonctionnel peut être utilisé en une quantité de 0,05 à 15% en poids, mieux de 0,1 à 10% en poids, et préférentiellement de 0,5% à 5% en poids de composé par rapport au poids total du copolymère final.

Le copolymère selon l'invention est susceptible d'être obtenu par un procédé comprenant :

- une première étape comprenant la polymérisation radicalaire en présence d'au moins un composé multifonctionnel comportant au moins deux groupes fonctionnels polymérisables, d'une partie des monomères envisagés, choisis parmi le/les monomères sélectionnés et/ou additionnels tels que définis ci-dessus; et
- une seconde étape comprenant la polymérisation radicalaire des monomères envisagés, choisis parmi le/les monomères sélectionnés et/ou additionnels tels que définis ci-dessus, éventuellement en présence de composé multifonctionnel.

La synthèse de copolymères hyperbranchés peut aussi être réalisée en faisant réagir:

- le ou les monomères éthyléniques envisagés, polymérisables par voie radicalaire, et

- des composés comportant au moins une insaturation et au moins un site capable d'amorcer la polymérisation, après activation par la température, le rayonnement UV ou selon tout autre mécanisme différent du mécanisme de polymérisation de l'insaturation; ces composés comportant donc à la fois un site capable de promouvoir la propagation des chaînes c'est à dire comportant une double liaison et un site A apte à amorcer les chaînes.

Cette méthode est nommée "self condensing vinyl polymerization". Elle est décrite dans l'ouvrage "Chimie et physicochimie des polymères" M. Fontanille, Y Gnanou (ed. DUNOD) 2002, page 368.

Pour former des polymères hyperbranchés, les insaturations ou/et le site A apte à amorcer les chaînes peuvent être capables de réagir selon un mécanisme de polymérisation radicalaire contrôlée. En particulier selon un procédé de polymérisation par transfert d'atome (ATRP), décrit notamment dans Macromolecules 2002, 35, pp.1146-48 Jho JY et Yoo SH; ou encore dans Polym. Prepr. 1996, 37(2), 413-14; ou bien Matyjaszewski "Progress in polymer science" 26 (3),337-77, 2001 Annexe 2.2; Chem review 2001, 101/12,3737 ; Mueller "Macromolecules" 34/18,62026-13, 2001.

D'une manière générale, la polymérisation radicalaire par transfert d'atomes s'effectue par polymérisation d'un ou de plusieurs monomères polymérisables par voie radicalaire, en présence :

- d'un amorceur ayant au moins un atome d'halogène transférable,
- d'un composé comprenant un métal de transition susceptible de participer à une étape de réduction avec l'amorceur et une chaîne polymérique "dormante";
- et d'un ligand pouvant être choisi parmi les composés comprenant un atome d'azote (N), d'oxygène (O), de phosphore (P) ou de soufre (S), susceptibles de se coordonner par une liaison audit composé comprenant un métal de transition. Ce procédé est en particulier décrit dans la demande WO 97/18247 et dans l'article de Matyjasezwski et al. publié dans JACS, 117, page 5614 (1995). L'atome d'halogène est de préférence un atome de chlore ou de brome. Dans ce cas, le site A apte à amorcer les chaînes pourra être par exemple R1-Cl avec R1= benzene, OCOCH(CH3)-.

Parmi les autres procédés utilisables, on peut citer la méthode des nitroxydes, décrite par exemple dans Chem review 2001,101(12) p 3681. La technique de polymérisation radicalaire par réaction avec un nitroxyde consiste à bloquer l'espèce radicalaire en croissance sous forme de liaison de type C-O NR1R2, R1 et R2 pouvant être, indépendamment l'un de l'autre, un radical alkyle ayant de 2 à 30 atomes de carbone ou formant l'un et l'autre, avec l'atome d'azote, un cycle ayant de 4 à 20 atomes de carbone, comme par exemple un cycle 2,2,6,6-tétraméthylpipéridinyle.

Cette technique de polymérisation est notamment décrite dans les articles "Synthesis of nitroxy-functionalized polybutadiène by anionic polymerization using a nitroxy-functionalized terminator", publié dans Macromolecules 1997, volume 30, pp.4238-42, et "Macromolecular engineering via living free radical polymerizations" publié dans Macromol. Chem. Phys. 1998, vol. 199, pp.923-935, ou bien encore dans la demande WO-A-99/03894.

Dans ce cas, le site A apte à amorcer les chaînes pourra être par exemple de formule :

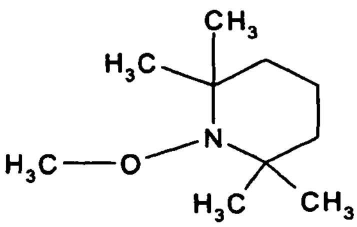

On peut également employer la méthode de polymérisation anionique décrite par exemple dans Chem. Review 2001, 101/12, p.3787, ainsi que la méthode de polymérisation cationique décrite par exemple par Charleux et al. Advances in Polymer Science 142, pp.1-69,1999.

Les copolymères selon l'invention présentent l'avantage de pouvoir facilement être mis en oeuvre dans les milieux cosmétiques organiques, notamment de type huileux ou solvant lipophile, tout en conservant des propriétés rhéologiques intéressantes.

Ils peuvent être présents dans les compositions cosmétiques ou pharmaceutiques, notamment dermatologiques topiques, en une quantité de 0,1 à 95% en poids, de préférence 0,5 à 90% en poids, notamment 1 à 80% en poids, voire 5-70% en poids, par rapport au poids total de la composition.

Les copolymères peuvent être présents dans la composition sous forme solubilisée, par exemple dans un solvant organique cosmétique ou une huile cosmétique. On a en effet constaté que les copolymères selon l'invention y sont tout particulièrement solubles et qu'ils peuvent y être mis en oeuvre tout en conservant des propriétés rhéologiques intéressantes.

Les compositions cosmétiques ou pharmaceutiques, notamment dermatologiques selon l'invention comprennent, outre lesdits copolymères, un milieu physiologiquement acceptable, notamment cosmétiquement ou dermatologiquement acceptable, c'est-à-dire un milieu compatible avec les matières kératiniques telles que la peau du visage ou du corps, les lèvres, les cheveux, les cils, les sourcils et les ongles.

La composition peut avantageusement comprendre un milieux solvant qui peut être une phase grasse, qui peut elle-même comprendre des huiles et/ou des solvants de préférence lipophiles, ainsi que des corps gras solides à température ambiante tels que les cires, des corps gras pâteux, des gommes, ainsi que leurs mélanges.

Parmi les constituants de la phase grasse, on peut préférentiellement citer les huiles et/ou solvants ayant un paramètre de solubilité global selon l'espace de solubilité de Hansen inférieur ou égal à 20 $(MPa)^{1/2}$, de préférence inférieur ou égal à 18 $(MPa)^{1/2}$, encore mieux inférieur ou égal à 17 $(MPa)^{1/2}$.

Le paramètre de solubilité global δ selon l'espace de solubilité de Hansen est défini dans l'article "Solubility parameter values" de Eric Grulke dans l'ouvrage "Polymer Handbook", 3éme édition, chapitre VII, p.519-559 par la relation :

$$\delta = (dD^2 + dP^2 + dH^2)^{1/2}$$

dans laquelle :

- dD caractérise les forces de dispersion de LONDON issues de la formation de dipôles induits lors des chocs moléculaires,
- dP caractérise les forces d'interactions de DEBYE entre dipôles permanents, et
- dH caractérise les forces d'interactions spécifiques (type liaisons hydrogène, acide/base, donneur/accepteur, etc.).

La définition des solvants dans l'espace de solubilité selon Hansen est décrite dans l'article de C.M.Hansen "The three dimensional solubility parameters" J.Paint Technol. 39, 105 (1967).

Parmi les huiles et/ou solvants ayant un paramètre de solubilité global selon l'espace de solubilité de Hansen inférieur ou égal à 20 $(MPa)^{1/2}$, on peut citer les huiles, volatiles ou non, qui peuvent être choisies parmi les huiles naturelles ou synthétiques, carbonées, hydrocarbonées, fluorées, éventuellement ramifiées, seules ou en mélange; les éthers et esters ayant plus de 6 atomes de carbone, notamment 6 à 30 atomes de carbone; les cétones ayant plus de 6 atomes de carbone, notamment 6 à 30 atomes de carbone; les monoalcools gras aliphatiques ayant 6 à 30 atomes de carbone, la chaîne hydrocarbonée ne comportant pas de groupement de substitution.

On entend par "huile non volatile", une huile susceptible de rester sur la peau à température ambiante et pression atmosphérique au moins une heure et ayant notamment une pression de vapeur à température ambiante (25°C) et pression atmosphérique, non nulle, inférieure à 0,01 mm de Hg (1,33 Pa).

On peut en particulier citer les huiles non volatile carbonées, notamment hydrocarbonées, d'origine végétale, minérale, animale ou synthétique, telles que l'huile de paraffine (ou vaseline), le squalane, le polyisobutylène hydrogéné (huile de Parléam), le perhydrosqualène, l'huile de vison, de macadamia, de tortue, de soja, l'huile d'amande douce, de calophyllum, de palme, de pépins de raisin, de sésame, de maïs, d'arara, de colza, de tournesol, de coton, d'abricot, de ricin, d'avocat, de jojoba, d'olive ou de germes de céréales, de beurre de karité; les esters linéaires, ramifiés ou cycliques, ayant plus de 6 atomes de carbone, notamment 6 à 30 atomes de carbone, tels que les esters d'acide lanolique, d'acide oléique, d'acide laurique, d'acide stéarique; les esters dérivés d'acides ou d'alcools à longue chaîne (c'est-à-dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, en particulier les esters en C12-C36, tels que le myristate d'isopropyle, le palmitate d'isopropyle, le stéarate de butyle, le laurate d'hexyle, l'adipate de diisopropyle, l'isononanoate d'isononyle, le palmitate de 2-éthylhexyle, le laurate de 2-hexyl-décyle, le palmitate de 2-octyl-décyle, le myristate ou le lactate de 2-octyl-dodécyle, le succinate de di(2-éthyl hexyle), le malate de diisostéaryle, le triisostéarate de glycérine ou de diglycérine; les acides gras supérieurs, notamment en C14-C22, tels que l'acide myristique, l'acide palmitique, l'acide stéarique, l'acide béhénique, l'acide oléique, l'acide linoléique, l'acide linolénique ou l'acide isostéarique; les alcools gras supérieurs, notamment en C16-C22, tels que le cétanol, l'alcool oléique, l'alcool linoléique ou linolénique, l'alcool isostéarique ou l'octyl dodécanol; et leurs mélanges.

On peut encore citer le décanol, le dodécanol, l'octadécanol, les triglycérides liquides d'acides gras de 4 à 10 atomes de carbone comme les triglycérides des acides heptanoïque ou octanoïque, les triglycérides des acides caprylique/caprique; les hydrocarbures linéaires ou ramifiés, d'origine minérale ou synthétique tels que les huiles de paraffine et leurs dérivés, la vaseline, les polydécènes, le polyisobutène hydrogéné tel que le parléam; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés

comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol.

On peut encore citer les cétones liquides à température ambiante tels que méthyléthylcétone, méthylisobutylcétone, diisobutylcétone, l'isophorone, la cyclohexanone, l'acétone ; les éthers de propylène glycol liquides à température ambiante tels que le monométhyléther de propylène glycol, l'acétate de monométhyléther de propylène glycol, le mono n-butyl éther de dipropylène glycol ; les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle; les éthers liquides à température ambiante tels que le diéthyléther, le diméthyléther ou le dichlorodiéthyléther; les alcanes liquides à température ambiante tels que le décane, l'heptane, le dodécane, l'isododécane, l'isohexadécane, le cyclohexane; les composés cycliques aromatiques liquides à température ambiante tels que le toluène et le xylène; les aldéhydes liquides à température ambiante tels que le benzaldéhyde, l'acétaldéhyde et leurs mélanges.

Parmi les composés volatils, on peut citer les huiles volatiles non siliconées, notamment les isoparaffines en C8-C16 comme l'isododécane, l'isodécane, l'isohexadécane et par exemple les huiles vendues sous les noms commerciaux d'ISOPAR, de PERMETHYL et notamment l'isododécane (PERMETHYL 99 A).

Plus préférentiellement, on peut citer les alcanes liquides à température ambiante, volatils ou non, et plus particulièrement le décane, l'heptane, le dodécane, l'isododécane, l'isohexadécane, le cyclohexane, l'isodécane, et leurs mélanges.

Ces huiles et/ou solvants peuvent être généralement présents en une teneur allant de 0,01 à 95%, de préférence de 0,1 à 90%, de préférence encore de 10 à 85% en poids, par rapport au poids total de la composition, et mieux de 30 à 80%.

La composition peut en outre comprendre, un milieu hydrophile comprenant de l'eau ou un mélange d'eau et de solvant(s) organique(s) hydrophile(s) comme les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le pentylène glycol, et les polyéthylène glycols, ou bien encore des éthers en $C_2$ et des aldéhydes en $C_2$-$C_4$ hydrophiles.

L'eau ou le mélange d'eau et de solvants organiques hydrophiles peut être présent dans la composition selon l'invention en une teneur allant de 0,1 à 80% en poids, par rapport au poids total de la composition, et de préférence de 1 à 70% en poids.

La composition selon l'invention peut également comprendre des cires et/ou des gommes.

Par cire au sens de la présente invention, on entend un composé lipophile, solide à température ambiante (25°C), à changement d'état solide/liquide réversible, ayant un point de fusion supérieur ou égal à 30°C pouvant aller jusqu'à 120°C. En portant la cire à l'état liquide (fusion), il est possible de la rendre miscible aux huiles éventuellement présentes et de former un mélange homogène microscopiquement, mais en ramenant la température du mélange à la température ambiante, on obtient une recristallisation de la cire dans les huiles du mélange. Le point de fusion de la cire peut être mesuré à l'aide d'un calorimètre à balayage différentiel (D.S.C.), par exemple le calorimètre vendu sous la dénomination DSC 30 par la société METTLER.

Les cires peuvent être hydrocarbonées, fluorées et/ou siliconées et être d'origine végétale, minérale, animale et/ou synthétique. En particulier, les cires présentent une température de fusion supérieure à 25°C et mieux supérieure à 45°C. Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.

La nature et la quantité des corps solides sont fonction des propriétés mécaniques et des textures recherchées. A titre indicatif, la composition peut contenir de 0,01 à 50% en poids de cires, par rapport au poids total de la composition et mieux de 1 à 30% en poids.

La composition selon l'invention peut en outre comprendre une ou des matières colorantes choisies parmi les colorants hydrosolubles, les colorants liposolubles, et les matières colorantes pulvérulentes comme les pigments, les nacres, et les paillettes bien connues de l'homme du métier. Les matières colorantes peuvent être présentes, dans la composition, en une teneur allant de 0,01 à 50% en poids, par rapport au poids de la composition, de préférence de 0,01 à 30% en poids.

Par pigments, il faut comprendre des particules de toute forme, blanches ou colorées, minérales ou organiques, insolubles dans le milieu physiologique, destinées à colorer la composition. Par nacres, il faut comprendre des particules de toute

forme irisées, notamment produites par certains mollusques dans leur coquille ou bien synthétisées. Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre. Parmi les pigments organiques, on peut citer le noir de carbone, les pigments de type D & C, et les laques à base de carmin de cochenille, de baryum, strontium, calcium, aluminium. Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.
Parmi les colorants hydrosolubles, on peut citer le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, le bleu de méthylène.

La composition selon l'invention peut comprendre en outre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition. Les charges peuvent être minérales ou organiques de toute forme, plaquettaires, sphériques ou oblongues, quelle que soit la forme cristallographique (par exemple feuillet, cubique, hexagonale, orthorhombique, etc.). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition peut comprendre en outre un polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques. Parmi les polymères filmogènes susceptibles d'être utilisés dans la composition de la présente invention, on peut citer les polymères synthétiques, de type radicalaire ou de type polycondensat, les polymères d'origine naturelle et leurs mélanges, en particulier les polymères acryliques, les polyuréthanes, les polyesters, les polyamides, les polyurées, les polymères cellulosiques comme la nitrocellulose.

La composition selon l'invention peut également comprendre des ingrédients couramment utilisés en cosmétique, tels que les vitamines, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, les céramides, ou leurs mélanges. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition selon l'invention peut se présenter notamment sous forme de suspension, de dispersion, de solution notamment organique, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de pâte, notamment de pâte souple (notamment de pâte ayant de viscosité dynamique à 25°C de l'ordre de 0,1 à 40 Pa.s sous une vitesse de cisaillement de 200 $s^{-1}$, après 10 minutes de mesure en géométrie cône/plan). La composition peut être anhydre : il peut s'agir d'une pâte anhydre.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition selon invention peut être une composition de maquillage, notamment un produit pour le teint tel qu'un fond de teint, un fard à joues ou à paupières; un produit pour les lèvres tel qu'un rouge à lèvres ou un soin des lèvres; un produit anti-cernes; un blush, un mascara, un eye-liner; un produit de maquillage des sourcils, un crayon à lèvres ou à yeux; un produit pour les ongles tel qu'un vernis à ongles ou un soin des ongles; un produit de maquillage du corps; un produit de maquillage des cheveux (mascara ou laque pour cheveux).

La composition selon l'invention peut être une composition de protection ou de soin de la peau du visage, du cou, des mains ou du corps, notamment une composition anti-rides, anti-fatigue permettant de donner un coup d'éclat à la peau, une composition hydratante ou traitante; une composition anti-solaire ou de bronzage artificiel.
La composition selon l'invention peut être également un produit capillaire, notamment pour le maintien de la coiffure ou la mise en forme des cheveux. Les compositions capillaires sont de préférence des shampooings, des gels, des lotions de mise en plis, des lotions pour le brushing, des compositions de fixation et de coiffage telles que les laques ou spray. Les lotions peuvent être conditionnées sous diverses formes, notamment dans des vaporisateurs, des flacons-pompe ou dans des récipients aérosol afin d'assurer une application de la composition sous forme vaporisée ou sous forme de mousse. De telles formes de conditionnement sont indiquées, par exemple lorsque l'on souhaite obtenir un spray, une mousse pour la fixation ou le traitement des cheveux.

Avantageusement, la composition selon l'invention peut être une composition de maquillage, notamment un fond de teint ou un rouge à lèvres.

L'invention a aussi pour objet un procédé cosmétique de maquillage ou de soin des matières kératiniques, notamment de la peau du corps ou du visage, des lèvres, des ongles, des cheveux et/ou des cils, comprenant l'application sur lesdites matières d'une composition cosmétique telle que définie précédemment.

En particulier, l'invention a pour objet un procédé cosmétique de maquillage de la peau du visage et/ou des lèvres, comprenant l'application sur lesdites matières d'une composition cosmétique de fond de teint ou de rouge à lèvres telle que définie précédemment.

L'invention est illustrée plus en détails dans les exemples suivants.

**Méthode de détermination du module dynamique de conservation E'**

La détermination du module dynamique de conservation E' s'effectue par DMTA (Dynamical and Mechanical Temperature Analysis ou Analyse dynamique et mécanique de la température).
Pour mesurer E', on effectue des essais de viscoélasticimétrie avec un appareil DMTA de TA Instruments (Modèle DMA2980) sur un échantillon de film de polymère d'environ 250±50 μm d'épaisseur, 5 mm de largeur et 10 mm de longueur, après séchage pendant 4 jours à 23°C et 50-55% d'humidité relative. On impose à cet échantillon une sollicitation en traction. L'échantillon subit une faible force statique (≅ 0,1N) à laquelle se superpose un déplacement sinusoïdal de ±8μm à la fréquence de 1 Hz. On travaille ainsi dans le domaine linéaire, sous de faibles niveaux de déformation.
Cette sollicitation de traction est effectuée sur l'échantillon à des températures variant de -150°C à +200°C, avec une variation de température de 3°C par minute.
On mesure alors à l'aide du DMA le module complexe E*=E' +iE du polymère testé (E' désignant le module dynamique de conservation et E désignant le module dynamique de perte).
De la valeur du module complexe à 22°C, on en déduit le module dynamique de conservation E' à une fréquence de 1 Hz.

**Exemple 1**

Dans un réacteur de 500 ml, avec agitation centrale, arrivée d'azote, thermomètre et réfrigérant, on introduit 75 g d'heptane et on chauffe à 90°C.
On introduit alors :

- coulée 1 : 35 g d'acrylate d'isobomyle, 34 g de méthacrylate d'isobomyle et 1 g de méthacrylate d'allyle, en 1 heure;
- coulée 2 : 1 g de Trigonox 21 S et 25 g d'heptane, en 48 heures.

Quand la coulée 1 est terminée, on introduit la coulée 3 comprenant : 30 g d'acrylate d'éthyl-2-hexyle, en 2 heures.
Le chauffage est constamment maintenu à 90°C. On laisse 3 heures au reflux du solvant, après la fin de la coulée 2.

On ajoute 120 g d'isododécane, puis on distille l'heptane sous pression réduite, et on récupère une solution du polymère dans l'isododécane, avec un extrait sec de 51%.

- Température de transition vitreuse : 10°C
- Mesure du module E' = 64 ± 3 MPA

**Exemple 2**

On prépare un fond de teint anhydre comprenant (% en poids) :

| | |
|---|---|
| - cire de polyéthylène | 12% |

(suite)

| | |
|---|---|
| - huiles siliconées volatiles | 25% |
| - phényltriméthicone | 20% |
| - Microsphères de polyméthylméthacrylate | 12% |
| - Solution du polymère de l'exemple 1 à 51% MS (matière sèche) dans l'isododécane | 12% |
| - Isododécane | qsp 100% |

On fait fondre les cires puis, quand tout est limpide, on ajoute la phényl triméthicone sous agitation, et les huiles de silicones; on ajoute ensuite les microsphères, l'isododécane et le polymère. On homogénéise pendant 15 minutes puis on coule la composition résultante que l'on laisse refroidir. On obtient un fond de teint anhydre.

**Exemple 3**

On prépare un stick de rouge à lèvres comprenant :

| | |
|---|---|
| - Cire de polyéthylène | 15% |
| - Solution du polymère de l'exemple 1 à 51% MS dans l'isododécane | 20% |
| - Polyisobutène hydrogéné (Parléam de Nippon Oil Fats) | 25% |
| - Pigments | 10% |
| - Isododécane | qsp 100% |

La composition obtenue après application sur les lèvres présente de bonnes propriétés cosmétiques.

**Exemple 4**

On prépare une composition de fond de teint comprenant (% en poids) :

Phase A

| | |
|---|---|
| - Cetyl Dimethicone copolyol (ABIL EM 90 de GOLDSCHMIDT) | 3 g |
| - Succinate d'isostéaryl diglycéryle (IMWITOR 780K de la société CONDEA) | 0,6 g |
| - Pigments (oxydes de fer et de titane) | 10 g |
| - Poudre de polyamide (NYLON-12) | 8 g |
| - Solution du polymère de l'exemple 1 à 51% MS dans l'isododécane | 17 g |
| - Parfum | qs |
| - Isododécane | 10 g |

Phase B

| | |
|---|---|
| - Sulfate de magnésium | 0,7 g |
| - Conservateur | qs |
| - Eau | qsp 100 g |

La composition obtenue présente de bonnes propriétés cosmétiques.

**Revendications**

1. Copolymère hyperbranché, comprenant au moins deux, notamment trois, branches polymériques, qui comportent,

chacune, au moins un point de ramification au moins trivalent, distinct et indépendant l'un de l'autre, dans lequel

- une première branche comprend au moins un premier monomère choisi parmi l'acrylate d'isobornyle et le méthacrylate d'isobornyle, et
- au moins une deuxième branche comprend au moins un deuxième monomère, identique ou différent dudit premier monomère, et choisi dans le groupe formé par l'acrylate d'isobornyle, le méthacrylate d'isobornyle, l'acrylate d'isobutyle, le méthacrylate d'isobutyle, et l'acrylate d'éthyle-2-hexyle.

**2.** Copolymère selon la revendication 1, dans lequel les branches sont reliées entre elles par un composé multifonctionnel, comportant au moins deux groupes fonctionnels polymérisables.

**3.** Copolymère selon la revendication 2, dans lequel le composé multifonctionnel répond à la formule suivante (I):

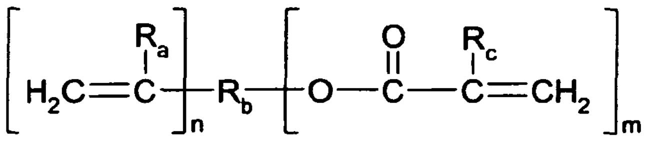

(I)

dans laquelle :

- Ra et Rc sont, indépendamment l'un de l'autre choisi parmi l'hydrogène ou les radicaux alkyles, linéaires ou ramifiés, ayant 1 à 22 atomes de carbone, notamment 1 à 3 atomes de carbone; de préférence Ra peut être un atome d'hydrogène; de préférence Rc peut être un radical méthyle;
- Rb est un groupe divalent alkylène linéaire ou ramifié, ayant 1 à 22 atomes de carbone, ou cycloalkylène ayant 3 à 6 atomes de carbone, ou arylène ayant 6 à 18 atomes de carbone, ou alkarylène ayant 7 à 24 atomes de carbone; de préférence Rb est un groupe méthylène;
- m et n sont, indépendamment l'un de l'autre, égaux à 1, 2, 3 ou 4, et tels que la somme m+n est supérieure ou égale à 2; de préférence m=n=1.

**4.** Copolymère selon la revendication 3, dans lequel le composé multifonctionnel est un (méth)acrylate d'allyle, et mieux le méthacrylate d'allyle.

**5.** Copolymère selon l'une des revendications précédentes, dans lequel chaque branche se présente sous la forme d'une séquence polymérique de nature homopolymère, ou copolymère statistique, alterné, bloc ou gradient.

**6.** Copolymère selon l'une des revendications précédentes, dans lequel chaque branche se présente sous la forme d'un homopolymère ou d'un copolymère statistique linéaire.

**7.** Copolymère selon l'une des revendications précédentes, dans lequel la branche principale se présente sous la forme d'un homopolymère ou d'un copolymère statistique comprenant 80-100% en poids, de préférence 100% en poids, par rapport au poids de la branche, de monomère(s) choisi(s) dans le groupe formé par l'acrylate d'isobornyle, le méthacrylate d'isobomyle, l'acrylate d'isobutyle, le méthacrylate d'isobutyle et l'acrylate d'éthyle-2-hexyle.

**8.** Copolymère selon l'une des revendications précédentes, dans lequel la/les branche(s) secondaire(s) se présente(nt) sous la forme d'un homopolymère ou d'un copolymère statistique comprenant 80-100% en poids, de préférence 100% en poids, par rapport au poids de la branche, de monomère(s) choisi(s) dans le groupe formé par l'acrylate d'isobornyle, le méthacrylate d'isobornyle, l'acrylate d'isobutyle, le méthacrylate d'isobutyle et l'acrylate d'éthyle-2-hexyle.

**9.** Copolymère selon l'une des revendications précédentes, dans lequel la quantité totale, dans le copolymère final, de monomère(s) choisi(s) dans le groupe formé par l'acrylate d'isobornyle, le méthacrylate d'isobomyle, l'acrylate d'isobutyle, le méthacrylate d'isobutyle et l'acrylate d'éthyle-2-hexyle, est comprise entre 50 et 100% en poids, notamment de 60 à 98% en poids, voire de 70 à 97% en poids, encore mieux de 80 à 96% en poids, préférentiellement de 90 à 95% en poids, par rapport au poids total de monomères présents dans le copolymère final.

**10.** Copolymère selon l'une des revendications précédentes, comprenant, de manière optionnelle, de 1 à 40% en poids,

notamment de 5 à 30% en poids, voire de 10 à 25% en poids, par rapport au poids du copolymère final, d'un troisième monomère choisi dans le groupe formé par l'acrylate d'isobornyle, le méthacrylate d'isobornyle, l'acrylate d'isobutyle, le méthacrylate d'isobutyle, et l'acrylate d'éthyle-2-hexyle, les % étant donnés par rapport au poids total de monomères présents dans le copolymère final.

**11.** Copolymère selon l'une des revendications précédentes, comprenant, dans la même branche ou dans des branches différentes, les associations de monomères suivants :

- de l'acrylate d'isobornyle et du méthacrylate d'isobornyle;
- de l'acrylate d'isobornyle et de l'acrylate d'isobutyle;
- de l'acrylate d'isobornyle et du méthacrylate d'isobutyle;
- de l'acrylate d'isobornyle et de l'acrylate d'éthyle-2-hexyle;
- du méthacrylate d'isobornyle et de l'acrylate d'isobutyle;
- du méthacrylate d'isobomyle et du méthacrylate d'isobutyle;
- du méthacrylate d'isobornyle et de l'acrylate d'éthyle-2-hexyle;
- de l'acrylate d'isobornyle, du méthacrylate d'isobornyle et de l'acrylate d'éthyle-2-hexyle;
- de l'acrylate d'isobornyle, du méthacrylate d'isobornyle et de l'acrylate d'isobutyle;
- de l'acrylate d'isobornyle, du méthacrylate d'isobutyle et de l'acrylate d'éthyle-2-hexyle;
- de l'acrylate d'isobomyle, du méthacrylate d'isobutyle et de l'acrylate d'isobutyle; et
- du méthacrylate d'isobornyle, du méthacrylate d'isobutyle et de l'acrylate d'isobutyle.

**12.** Copolymère selon l'une des revendications précédentes, comprenant,

- de l'acrylate d'isobomyle dans les branches principales et de l'acrylate d'isobutyle dans les branches secondaires;
- de l'acrylate d'isobornyle dans les branches principales et de l'acrylate d'éthyle-2-hexyle dans les branches secondaires;
- du méthacrylate d'isobornyle dans les branches principales et de l'acrylate d'isobutyle dans les branches secondaires;
- du méthacrylate d'isobomyle dans les branches principales et de l'acrylate d'éthyle-2-hexyle dans les branches secondaires;
- de l'acrylate d'isobomyle et du méthacrylate d'isobornyle dans les branches principales et de l'acrylate d'isobutyle dans les branches secondaires;
- de l'acrylate d'isobomyle et du méthacrylate d'isobomyle dans les branches principales et de l'acrylate d'éthyle-2-hexyle dans les branches secondaires;
- de l'acrylate d'isobornyle et du méthacrylate d'isobutyle dans les branches principales et de l'acrylate d'isobutyle dans les branches secondaires;
- de l'acrylate d'isobornyle et du méthacrylate d'isobutyle dans les branches principales et de l'acrylate d'éthyle-2-hexyle dans les branches secondaires;
- du méthacrylate d'isobornyle et du méthacrylate d'isobutyle dans les branches principales et de l'acrylate d'isobutyle dans les branches secondaires; ou
- du méthacrylate d'isobomyle et du méthacrylate d'isobutyle dans les branches principales et de l'acrylate d'éthyle-2-hexyle dans les branches secondaires.

**13.** Copolymère selon l'une des revendications précédentes, comprenant en outre au moins un monomère additionnel, qui peut être présent en une quantité de 0 à 50% en poids, notamment de 2 à 40% en poids, voire de 3 à 30% en poids, encore mieux de 4 à 20% en poids, préférentiellement de 5 à 10% en poids, par rapport au poids total de monomères présents dans le copolymère final.

**14.** Copolymère selon la revendication 13, dans lequel le monomère additionnel est choisi parmi, seul ou en mélange, les monomères suivants, ainsi que leurs sels:

- (i) les (méth)acrylates de formule $CH_2$=CHCOOR ou $CH_2$=C($CH_3$)COOR dans laquelle R représente :

  - un groupe alkyle linéaire ou ramifié, comprenant 1 à 30 atomes de carbone dans lequel se trouve(nt) éventuellement intercalé(s) un ou plusieurs hétéroatomes choisis parmi O, N, S et P et/ou ledit groupe alkyle pouvant être éventuellement substitué par un ou plusieurs substituants choisis parmi -OH, les atomes d'halogène (Cl, Br, I et F), les groupes -NR4R5, où R4 et R5 identiques ou différents représentent l'hydrogène

ou un groupe alkyle linéaire ou ramifié, en C1 à C6 ou un groupe phényle; et/ou les groupes polyoxyalkylènes, notamment polyoxyéthylène et/ou polyoxypropylène, ledit groupe polyoxyalkylène étant constitué par la répétition de 5 à 30 motifs oxyalkylène;
- un groupe cycloalkyle en C3 à C12, ledit groupe cycloalkyle pouvant comporter dans sa chaîne un ou plusieurs hétéroatomes choisis parmi O, N, S et/ou P, et/ou pouvant être éventuellement substitué par un ou plusieurs substituants choisis parmi -OH et les atomes d'halogène (Cl, Br, I et F);
- un groupe aryle en C4 à C20 ou aralkyle en C5 à C30 (groupe alkyle en C1 à C8);

notamment R peut être un groupe méthyle, éthyle, propyle, isopropyle, n-butyle, isobutyle, tertiobutyle, hexyle, éthylhexyle, octyle, lauryle, isooctyle, isodécyle, dodécyle, cyclohexyle, t-butylcyclohexyle, stéaryle, éthyl-2-perfluorohexyle, 2-hydroxyéthyle, 2-hydroxybutyle, 2-hydroxypropyle, méthoxyéthyle, éthoxyéthyle, méthoxy-propyle, isobornyle, phényle, 2-phényl-éthyle, t-butylbenzyle, benzyle, furfurylméthyle ou tétrahydrofurfurylméthyle, méthoxy-polyoxyéthylène (ou POE-méthyle); POE-béhényle, trifluoroéthyle; diméthylaminoéthyle, diéthylaminoéthyle, diméthylaminopropyle.
- (ii) les (méth)acrylamides de formule $CH_2{=}CHCONR_4R_5$ ou $CH_2{=}C(CH_3)CONR_4R_5$
dans laquelle R4 et R5, identiques ou différents, représentent un atome d'hydrogène ou :

a) un groupe alkyle, linéaire ou ramifié, comportant de 1 à 18 atomes de carbone, dans lequel se trouve (nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (Cl, Br, I et F), et les groupes Si (R4R5), où R4 et R5 identiques ou différents représentent un groupe alkyle en C1 à C6 ou un groupe phényle ;
et notamment un groupe méthyle, éthyle, propyle, isopropyle, n-butyle, isobutyle, tertiobutyle, hexyle, isohexyle, cyclohexyle, éthylhexyle, octyle, isooctyle, décyle, isodécyle, cyclodécyle, dodécyle, cyclododécyle, isononyle, lauryle, t-butylcyclohexyle, stéaryle; éthyl-2-perfluorohexyle; ou un groupe hydroxyalkyle en C1-4 tel que 2-hydroxyéthyle, 2-hydroxybutyle et 2-hydroxypropyle; ou un groupe alcoxy(C1-4)-alkyle(C1-4) tel que méthoxyéthyle, éthoxyéthyle et méthoxy-propyle,
b) un groupe cycloalkyle en C3 à C12, tel que le groupe isobornyle, ou un groupe hétérocycloalkyle (alkyle de 1 à 4 atomes de carbone), tel que furfurylméthyle ou tétrahydrofurfurylméthyle,
c) un groupe aryle en C4 à C20 tel que le groupe phényle,
d) un groupe aralkyle en C5 à C30 (groupe alkyle en C1 à C8) tel que 2-phényl éthyle, t-butylbenzyle ou benzyle,

- (iii) les monomères à insaturation(s) éthylénique(s) comprenant au moins une fonction acide carboxylique, phosphorique ou sulfonique, ou anhydride, comme par exemple l'acide acrylique, l'acide méthacrylique, l'acide crotonique, l'anhydride maléique, l'acide itaconique, l'acide fumarique, l'acide maléique, l'acide styrènesulfonique, l'acide vinylbenzoïque, l'acide vinylphosphorique, l'acide acrylamidopropanesulfonique; et les sels de ceux ci;
- (iv) les éthers de vinyle de formule $R_6O{-}CH{=}CH_2$ ou les esters de vinyle de formule : $R_6{-}COO{-}CH{=}CH_2$
dans laquelle $R_6$ représente un groupe alkyle linéaire ou ramifié, comprenant de 1 à 22 atomes, ou un groupe alkyle cyclique comportant de 3 à 6 atomes de carbone et/ou un groupe aromatique, par exemple de type benzénique, anthracénique, et naphtalénique ;
- (v) les composés vinyliques de formules : CH2=CH-R9, CH2=CH-CH2-R9 ou CH2=C(CH3)-CH2-R9
dans lesquelles R9 est un groupe hydroxyle, halogène (Cl ou F), NH2, OR10 où R10 représente un groupe phényle ou un groupe alkyle en C1 à C12 (le monomère est un éther de vinyle ou d'allyle); acétamide (NHCOCH3); un groupe OCOR11 où R11 représente un groupe alkyle de 2 à 12 carbones, linéaire ou ramifié (le monomère est un ester de vinyle ou d'allyle); ou un groupe choisi parmi :

- un groupe alkyle linéaire ou ramifié, de 1 à 18 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi les groupes hydroxyle, les atomes d'halogène (Cl, Br, I et F), et les groupes Si (R4R5), où R4 et R5 identiques ou différents représentent un groupe alkyle en C1 à C6 ou un groupe phényle ;
- un groupe cycloalkyle en C3 à C12 tel que isobornyle, cyclohexane,
- un groupe aryle en C3 à C20 tel que phényle,
- un groupe aralkyle en C4 à C30 (groupe alkyle en C1 à C8) tel que 2-phényléthyle ; benzyle,
- un groupe hétérocyclique de 4 à 12 chaînons contenant un ou plusieurs hétéroatomes choisis parmi O, N, et S, le cycle étant aromatique ou non,

- un groupe hétérocycloalkyle (alkyle de 1 à 4 C), tel que furfurylméthyle ou tétrahydrofurfurylméthyle,

- (vi) le styrène et ses dérivés, notamment tels que le méthylstyrène, le chlorostyrène ou le chlorométhylstyrène;
- (vii) les monomères à insaturation éthylénique comprenant un ou plusieurs atomes de silicium tels que le méthacryloxypropyl triméthoxy silane, le méthacryloxypropyl tris(triméthylsiloxy)silane;
- ainsi que leurs sels et leurs mélanges.

**15.** Copolymère selon l'une des revendications 13 à 14, dans lequel le monomère additionnel est choisi parmi les (méth) acrylates de méthyle, d'éthyle, de n-propyle, d'isopropyle, de n-butyle, de t-butyle, de cyclohexyle, de méthoxyéthyle, d'éthoxyéthyle, de trifluoroéthyle, de diméthylaminoéthyle, de diéthylaminoéthyle, de 2-hydroxypropyle, de 2-hydroxyéthyle; l'acide acrylique, l'acide méthacrylique, le (méth)acrylamide, le méthacryloxypropyl triméthoxy silane, le méthacryloxypropyl tris( triméthylsiloxy)silane; ainsi que leurs sels; et leurs mélanges.

**16.** Copolymère selon l'une des revendications 13 à 15, dans lequel le monomère additionnel est choisi parmi des macromonomères carbonés ou siliconés ayant au moins un groupe terminal polymérisable, notamment un groupe vinyle ou (méth)acrylate.

**17.** Copolymère selon la revendication 16, dans lequel le macromonomère additionnel est choisi parmi, seuls ou en mélange, ainsi que leurs sels, :

(i) les homopolymères et les copolymères (méth)acrylate d'alkyle linéaire ou ramifié en C8-C22, présentant un groupe terminal polymérisable choisi parmi les groupes vinyle ou (méth)acrylate parmi lesquels on peut citer les macromonomères de poly(acrylate d'éthyl-2 hexyle) à extrémité mono(méth)acrylate ; les macromonomères de poly(acrylate de dodécyle) ou de poly(méthacrylate de dodécyle) à extrémité mono(méth)acrylate; les macromonomères de poly(acrylate de stéaryle) ou de poly (méthacrylate de stéaryle) à extrémité mono(méth)acrylate.

- (ii) les polyoléfines ayant un groupe terminal à insaturation éthylénique, en particulier ceux ayant un groupement terminal (méth)acrylate; et notamment les macromonomères suivants, étant entendu qu'ils ont un groupe terminal (méth)acrylate: les macromonomères de polyéthylène, les macromonomères de polypropylène, les macromonomères de copolymère polyéthylène/polypropylène, les macromonomères de copolymère polyéthylène/polybutylène, les macromonomères de polyisobutylène; les macromonomères de polybutadiène; les macromonomères de polyisoprène; les macromonomères de polybutadiène; les macromonomères de poly(éthylène/butylène)-polyisoprène.
- (iii) les polydiméthylsiloxanes à groupement terminal mono (méth) acrylate, et notamment ceux de formule (IIa) suivante :

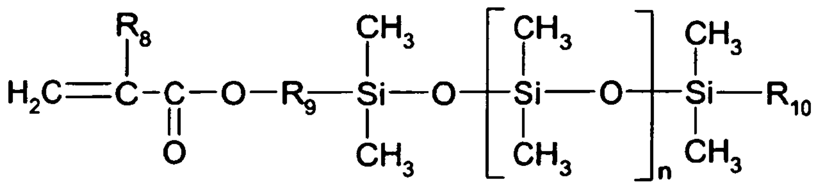

(IIa)

dans laquelle :

- $R_8$ désigne un atome d'hydrogène ou un groupement méthyle; de préférence méthyle;
- $R_9$ désigne un groupe hydrocarboné divalent, linéaire ou ramifié, de préférence linéaire, ayant de 1 à 10 atomes de carbone et contenant éventuellement une ou deux liaisons éther -O- ; de préférence éthylène, propylène ou butylène;
- $R_{10}$ désigne un groupe alkyle linéaire ou ramifié, ayant de 1 à 10 atomes de carbone, notamment de 2 à 8 atomes de carbone; de préférence méthyle, éthyle, propyle, butyle ou pentyle;
- n désigne un nombre entier allant de 1 à 300, de préférence allant de 3 à 200, et préférentiellement allant de 5 à 100.

**18.** Copolymère selon l'une des revendications précédentes, comprenant au moins une séquence polymérique ou branche dite "de Tg haute", c'est-à-dire ayant une Tg supérieure ou égale à 20°C, notamment comprise entre 20°C

et 150°C inclus, notamment comprise entre 30 et 120°C, plus particulièrement comprise entre 40°C et 100°C.

**19.** Copolymère selon la revendication 18, dans lequel la branche de Tg haute est présente en une quantité supérieure ou égale à 50%, notamment de 55% à 95%, préférentiellement de 60% à 90% en poids, par rapport au poids total du polymère.

**20.** Copolymère selon l'une des revendications précédentes, comprenant au moins une séquence ou branche dite "de Tg basse", c'est-à-dire ayant une Tg strictement inférieure à 20°C, c'est-à-dire comprise entre -150°C et 19°C, notamment comprise entre -100°C et 0°C, plus particulièrement comprise entre - 80°C et -5°C, voire comprise entre -70°C et -10°C.

**21.** Copolymère selon la revendication 20, dans lequel la branche de Tg basse est présente en une quantité inférieure à 50%, notamment de 5% à 45%, préférentiellement de 10% à 40% en poids, par rapport au poids total du polymère.

**22.** Copolymère selon l'une des revendications 18 à 19, dans lequel la branche de Tg haute comprend des monomères de Tg haute présents à raison de 30% à 100% en poids, notamment 40 à 95% en poids, encore mieux de 50 à 90% en poids, par rapport au poids total de ladite branche.

**23.** Copolymère selon l'une des revendications 20 à 21, dans lequel la branche de Tg basse comprend des monomères de Tg basse présents à raison de 30% à 100% en poids, notamment 40 à 95% en poids, encore mieux de 50 à 90% en poids, par rapport au poids total de ladite branche.

**24.** Copolymère selon l'une des revendications 18 à 23, dans lequel la branche principale est "de Tg haute" et au moins une des branches secondaires est "de Tg basse".

**25.** Copolymère selon l'une des revendications précédentes, comprenant au moins un monomère dit "de Tg haute", c'est-à-dire ayant une Tg supérieure ou égale à 20°C, notamment comprise entre 20 et 150°C, plus particulièrement comprise entre 30 et 120°C, et encore mieux comprise entre 40 et 100°C, ou un mélange de tels monomères.

**26.** Copolymère selon la revendication 25, dans lequel le ou les monomères dits "de Tg haute" sont présents à raison de 40 à 100% en poids, notamment 50 à 80% en poids, encore mieux 55 à 70% en poids, par rapport au poids total de monomères présents dans le copolymère.

**27.** Copolymère selon l'une des revendications précédentes, comprenant au moins un monomère dit "de Tg basse", c'est-à-dire ayant une Tg strictement inférieure à 20°C, notamment comprise entre -150 et 19°C, plus particulièrement comprise entre -100 et 0°C, encore mieux comprise entre -80 et -5°C, voire comprise entre -70°C et -10°C, ou un mélange de tels monomères.

**28.** Copolymère selon la revendication 27, dans lequel le/les monomères de Tg basse sont présent(s) à raison de 0 à 60% en poids, notamment de 20 à 50% en poids, encore mieux de 30 à 45% en poids, par rapport au poids total de monomères présents dans le copolymère.

**29.** Copolymère selon l'une des revendications précédentes, comprenant au moins un monomère lipophile, qui peut être présent à raison de 40 à 100% en poids, notamment 50 à 80% en poids, encore mieux 55 à 70% en poids, par rapport au poids total de monomères présents dans le copolymère.

**30.** Copolymère selon l'une des revendications précédentes, comprenant au moins un monomère hydrophile, qui peut être présent à raison de 0 à 60% en poids, notamment 20 à 50% en poids, encore mieux 30 à 45% en poids, par rapport au poids total de monomères présents dans le copolymère.

**31.** Copolymère selon l'une des revendications précédentes, dans lequel la branche principale est lipophile, et mieux dans lequel l'ensemble des branches, principale et secondaires, sont lipophiles.

**32.** Copolymère selon l'une des revendications précédentes, présentant une masse moléculaire moyenne en nombre comprise entre 2000 g/mol et 1 500 000 g/mol, notamment entre 5500 g/mol et 1 000 000 g/mol, et encore mieux entre 6000 g/mol et 900 000 g/mol.

**33.** Copolymère selon l'une des revendications précédentes, soluble à une concentration d'au moins 3% en poids, dans

l'isododécane, à 25°C, 1 atm., de préférence à une concentration d'au moins 5% en poids, voire d'au moins 10% en poids.

**34.** Composition cosmétique ou pharmaceutique, notamment dermatologique topique, comprenant, dans un milieu physiologiquement acceptable, notamment cosmétiquement ou dermatologiquement acceptable, au moins un copolymère hyperbranché comprenant au moins deux, notamment trois, branches polymériques, qui comportent, chacune, au moins un point de ramification au moins trivalent, distinct et indépendant l'un de l'autre,
dans lequel une première branche comprend au moins un premier monomère choisi dans le groupe formé par l'acrylate d'isobornyle, le méthacrylate d'isobornyle, l'acrylate d'isobutyle, le méthacrylate d'isobutyle et l'acrylate d'éthyle-2-hexyle, et au moins une deuxième branche comprend au moins un deuxième monomère, identique ou différent dudit premier monomère, et choisi dans ledit groupe.

**35.** Composition selon la revendication 34, dans laquelle le copolymère est présent en une quantité de 0,1 à 95% en poids, de préférence 0,5 à 90% en poids, notamment 1 à 80% en poids, voire 5-70% en poids, par rapport au poids total de la composition.

**36.** Composition selon l'une des revendications 34 à 35, comprenant au moins une phase grasse, qui comprend au moins un constituant choisi parmi les huiles, les solvants de préférence lipophiles, les corps gras solides à température ambiante tels que les cires, les corps gras pâteux, les gommes, et leurs mélanges.

**37.** Composition selon la revendication 36, comprenant au moins un constituant choisi parmi les huiles et/ou solvants ayant un paramètre de solubilité global selon l'espace de solubilité de Hansen inférieur ou égal à 20 $(MPa)^{1/2}$, de préférence inférieur ou égal à 18 $(MPa)^{1/2}$, encore mieux inférieur ou égal à 17 $(mPa)^{1/2}$.

**38.** Composition selon la revendication 37, dans laquelle les huiles et/ou solvants ayant un paramètre de solubilité global selon l'espace de solubilité de Hansen inférieur ou égal à 20 $(MPa)^{1/2}$, sont choisis parmi les huiles, volatiles ou non, qui peuvent être choisies parmi les huiles naturelles ou synthétiques, carbonées, hydrocarbonées, fluorées, éventuellement ramifiées, seules ou en mélange; les éthers et esters ayant plus de 6 atomes de carbone, notamment 6 à 30 atomes de carbone; les cétones ayant plus de 6 atomes de carbone, notamment 6 à 30 atomes de carbone; les monoalcools gras aliphatiques ayant 6 à 30 atomes de carbone, la chaîne hydrocarbonée ne comportant pas de groupement de substitution.

**39.** Composition selon la revendication 38, dans laquelle les huiles et/ou solvants sont choisis parmi les huiles non volatile carbonées, notamment hydrocarbonées, d'origine végétale, minérale, animale ou synthétique, telles que l'huile de paraffine (ou vaseline), le squalane, le polyisobutylène hydrogéné (huile de Parléam), le perhydrosqualène, l'huile de vison, de macadamia, de tortue, de soja, l'huile d'amande douce, de calophyllum, de palme, de pépins de raisin, de sésame, de maïs, d'arara, de colza, de tournesol, de coton, d'abricot, de ricin, d'avocat, de jojoba, d'olive ou de germes de céréales, de beurre de karité; les esters linéaires, ramifiés ou cycliques, ayant plus de 6 atomes de carbone, notamment 6 à 30 atomes de carbone, tels que les esters d'acide lanolique, d'acide oléique, d'acide laurique, d'acide stéarique; les esters dérivés d'acides ou d'alcools à longue chaîne (c'est-à-dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, en particulier les esters en C12-C36, tels que le myristate d'isopropyle, le palmitate d'isopropyle, le stéarate de butyle, le laurate d'hexyle, l'adipate de diisopropyle, l'isononanoate d'isononyle, le palmitate de 2-éthylhexyle, le laurate de 2-hexyl-décyle, le palmitate de 2-octyl-décyle, le myristate ou le lactate de 2-octyl-dodécyle, le succinate de di(2-éthyl hexyle), le malate de diisostéaryle, le triisostéarate de glycérine ou de diglycérine; les acides gras supérieurs, notamment en C14-C22, tels que l'acide myristique, l'acide palmitique, l'acide stéarique, l'acide béhénique, l'acide oléique, l'acide linoléique, l'acide linolénique ou l'acide isostéarique; les alcools gras supérieurs, notamment en C16-C22, tels que le cétanol, l'alcool oléique, l'alcool linoléique ou linolénique, l'alcool isostéarique ou l'octyl dodécanol; et leurs mélanges; le décanol, le dodécanol, l'octadécanol, les triglycérides liquides d'acides gras de 4 à 10 atomes de carbone comme les triglycérides des acides heptanoïque ou octanoïque, les triglycérides des acides caprylique/caprique; les hydrocarbures linéaires ou ramifiés, d'origine minérale ou synthétique tels que les huiles de paraffine et leurs dérivés, la vaseline, les polydécènes, le polyisobutène hydrogéné tel que le parléam; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras; des esters de polyol comme le dioctanoate de propylène glycol, le diheptanoate de néopentylglycol, le diisononanoate de diéthylèneglycol; et les esters du pentaérythritol; des alcools gras ayant de 12 à 26 atomes de carbone comme l'octyldodécanol, le 2-butyloctanol, le 2-hexyldécanol, le 2-undécylpentadécanol; les cétones liquides à température ambiante tels que méthyléthylcétone, méthylisobutylcétone, diisobutylcétone, l'isophorone, la cyclohexanone, l'acétone ; les éthers de propylène glycol liquides à température ambiante tels que le monométhyléther de propylène glycol, l'acétate de monométhyléther de propylène glycol, le mono n-butyl éther de dipropylène glycol ; les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle; les éthers liquides à température ambiante tels que le diéthyléther, le diméthyléther ou le dichlorodiéthyléther; les alcanes liquides à température ambiante tels que le décane, l'heptane, le dodécane, l'isododécane, l'isohexadécane, le cyclohexane; les composés cycliques aromatiques liquides à température ambiante tels que le toluène et le xylène; les aldéhydes liquides à température ambiante tels que le benzaldéhyde, l'acétaldéhyde et leurs mélanges; les huiles volatiles non siliconées, notamment les isoparaffines en C8-C16 comme l'isododécane, l'isodécane, l'isohexadécane.

**40.** Composition selon l'une des revendications 34 à 39, comprenant au moins une huile et/ou solvant choisie parmi les alcanes liquides à température ambiante, volatils ou non, et plus particulièrement le décane, l'heptane, le dodécane, l'isododécane, l'isohexadécane, le cyclohexane, l'isodécane, et leurs mélanges.

**41.** Composition selon l'une des revendications 34 à 40, comprenant au moins de 0,01 à 95%, de préférence de 0,1 à 90%, de préférence encore de 10 à 85% en poids, par rapport au poids total de la composition, et mieux de 30 à 80%, d'au moins une huile et/ou solvant.

**42.** Composition selon l'une des revendications 34 à 41, comprenant en outre un constituant choisi parmi un milieu hydrophile comprenant de l'eau ou un mélange d'eau et de solvant organique hydrophile; les cires, les gommes; les matières colorantes; les charges; les polymères notamment filmogènes; les vitamines, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, les céramides, ou leurs mélanges.

**43.** Composition selon l'une des revendications 34, à 42, se présentant sous forme de suspension, de dispersion, de solution notamment organique, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphasé ou multiphase, de spray, de poudre, de pâte, notamment de pâte souple; sous forme anhydre, par exemple de pâte anhydre.

**44.** Composition selon l'une des revendications 34 à 43, se présentant sous forme d'une composition de maquillage, notamment un produit pour le teint tel qu'un fond de teint, un fard à joues ou à paupières; un produit pour les lèvres tel qu'un rouge à lèvres ou un soin des lèvres; un produit anti-cernes; un blush, un mascara, un eye-liner; un produit de maquillage des sourcils, un crayon à lèvres ou à yeux; un produit pour les ongles tel qu'un vernis à ongles ou un soin des ongles; un produit de maquillage du corps; un produit de maquillage des cheveux (mascara ou laque pour cheveux); d' une composition de protection ou de soin de la peau du visage, du cou, des mains ou du corps, notamment une composition anti-rides, anti-fatigue permettant de donner un coup d'éclat à la peau, une composition hydratante ou traitante; une composition anti-solaire ou de bronzage artificiel; d'une composition capillaire, notamment pour le maintien de la coiffure ou la mise en forme des cheveux.

**45.** Composition selon la revendication 44, se présentant sous forme d'une composition de maquillage, notamment un fond de teint ou un rouge à lèvres.

**46.** Procédé cosmétique de maquillage ou de soin des matières kératiniques, notamment de la peau du corps ou du visage, des lèvres, des ongles, des cheveux et/ou des cils, comprenant l'application sur lesdites matières d'une composition cosmétique telle que définie à l'une des revendications 34 à 45.

**47.** Procédé cosmétique de maquillage de la peau du visage et/ou des lèvres, comprenant l'application sur lesdites matières d'une composition cosmétique de fond de teint ou de rouge à lèvres telle que définie à la revendication 46.

## Claims

**1.** Hyperbranched copolymer comprising at least two, especially three, polymeric branches, each of which contains at

least one at least trifunctional branch point, different from and independent of one another, wherein

- a first branch contains at least one first monomer selected from isobornyl acrylate or isobornyl methacrylate, and
- at least one second branch contains at least one second monomer, identical to or different from said first monomer, selected from the group consisting of isobornyl acrylate, isobornyl methacrylate, isobutyl acrylate, isobutyl methacrylate and 2-ethylhexyl acrylate.

**2.** Copolymer according to Claim 1, wherein the branches are connected to one another by a polyfunctional compound containing at least two polymerizable functional groups.

**3.** Copolymer according to Claim 2, wherein the polyfunctional compound corresponds to the formula (I) below:

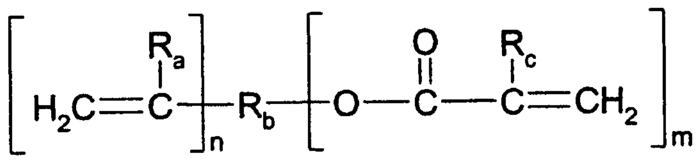

(I)

in which:

- $R_a$ and $R_c$ are selected independently of one another from hydrogen or linear or branched alkyl radicals having 1 to 22 carbon atoms, especially 1 to 3 carbon atoms; preferably $R_a$ may be a hydrogen atom; preferably $R_c$ may be a methyl radical;
- $R_b$ is a linear or branched divalent alkylene group having 1 to 22 carbon atoms, or a divalent cycloalkylene group having 3 to 6 carbon atoms, or a divalent arylene group having 6 to 18 carbon atoms, or a divalent alkarylene group having 7 to 24 carbon atoms; preferably $R_b$ is a methylene group;
- m and n independently of one another are 1, 2, 3 or 4 and are such that the sum m+n is greater than or equal to 2; preferably m=n=1.

**4.** Copolymer according to Claim 3, wherein the polyfunctional compound is an allyl (meth)acrylate and more preferably allyl methacrylate.

**5.** Copolymer according to one of the preceding claims, wherein each branch is in the form of a polymeric sequence of homopolymer type or of random, alternating, block or gradient copolymer type.

**6.** Copolymer according to one of the preceding claims, wherein each branch is in the form of a linear random copolymer or homopolymer.

**7.** Copolymer according to one of the preceding claims, wherein the main branch is in the form of a homopolymer or a random copolymer containing 80%-100% by weight, preferably 100% by weight, relative to the weight of the branch, of one or more monomers selected from the group consisting of isobornyl acrylate, isobornyl methacrylate, isobutyl acrylate, isobutyl methacrylate and 2-ethylhexyl acrylate.

**8.** Copolymer according to one of the preceding claims, wherein the secondary branch or branches is or are in the form of a homopolymer or a random copolymer containing 80%-100% by weight, preferably 100% by weight, relative to the weight of the branch, of one or more monomers selected from the group consisting of isobornyl acrylate, isobornyl methacrylate, isobutyl acrylate, isobutyl methacrylate and 2-ethylhexyl acrylate.

**9.** Copolymer according to one of the preceding claims, wherein the total amount, in the final copolymer, of one or more monomers selected from the group consisting of isobornyl acrylate, isobornyl methacrylate, isobutyl acrylate, isobutyl methacrylate and 2-ethylhexyl acrylate, is between 50% and 100% by weight, especially from 60% to 98% by weight, or even from 70% to 97% by weight, more preferably from 80% to 96% by weight, preferentially from 90% to 95% by weight, relative to the total weight of monomers present in the final copolymer.

**10.** Copolymer according to one of the preceding claims, containing, optionally, from 1% to 40% by weight, especially from 5% to 30% by weight, or even from 10% to 25% by weight, relative to the weight of the final copolymer, of a

third monomer selected from the group consisting of isobornyl acrylate, isobornyl methacrylate, isobutyl acrylate, isobutyl methacrylate and 2-ethylhexyl acrylate, the percentages being given relative to the total weight of monomers present in the final copolymer.

**11.** Copolymer according to one of the preceding claims, containing, in the same branch or in different branches, the following monomer combinations:

- isobornyl acrylate and isobornyl methacrylate;
- isobornyl acrylate and isobutyl acrylate;
- isobornyl acrylate and isobutyl methacrylate;
- isobornyl acrylate and 2-ethylhexyl acrylate;
- isobornyl methacrylate and isobutyl acrylate;
- isobornyl methacrylate and isobutyl methacrylate;
- isobornyl methacrylate and 2-ethylhexyl acrylate;
- isobornyl acrylate, isobornyl methacrylate and 2-ethylhexyl acrylate;
- isobornyl acrylate, isobornyl methacrylate and isobutyl acrylate;
- isobornyl acrylate, isobutyl methacrylate and 2-ethylhexyl acrylate;
- isobornyl acrylate, isobutyl methacrylate and isobutyl acrylate; and
- isobornyl methacrylate, isobutyl methacrylate and isobutyl acrylate.

**12.** Copolymer according to one of the preceding claims, containing

- isobornyl acrylate in the main branches and isobutyl acrylate in the secondary branches;
- isobornyl acrylate in the main branches and 2-ethylhexyl acrylate in the secondary branches;
- isobornyl methacrylate in the main branches and isobutyl acrylate in the secondary branches;
- isobornyl methacrylate in the main branches and 2-ethylhexyl acrylate in the secondary branches;
- isobornyl acrylate and isobornyl methacrylate in the main branches and isobutyl acrylate in the secondary branches;
- isobornyl acrylate and isobornyl methacrylate in the main branches and 2-ethylhexyl acrylate in the secondary branches;
- isobornyl acrylate and isobutyl methacrylate in the main branches and isobutyl acrylate in the secondary branches;
- isobornyl acrylate and isobutyl methacrylate in the main branches and 2-ethylhexyl acrylate in the secondary branches;
- isobornyl methacrylate and isobutyl methacrylate in the main branches and isobutyl acrylate in the secondary branches; or
- isobornyl methacrylate and isobutyl methacrylate in the main branches and 2-ethylhexyl acrylate in the secondary branches.

**13.** Copolymer according to one of the preceding claims, further comprising at least one additional monomer, which may be present in an amount of 0 to 50% by weight, especially from 2% to 40% by weight, or even from 3% to 30% by weight, more preferably from 4% to 20% by weight, preferentially from 5% to 10% by weight, relative to the total weight of monomers present in the final copolymer.

**14.** Copolymer according to Claim 13, wherein the additional monomer is selected, alone or in a mixture, from the following monomers, and also their salts:

- (i) (meth) acrylates of formula $CH_2$=CHCOOR or $CH_2$=C($CH_3$)COOR in which R represents:

  - a linear or branched alkyl group containing 1 to 30 carbon atoms, in which there is or are optionally intercalated one or more heteroatoms selected from O, N, S and P, and/or it being possible for said alkyl group to be optionally substituted by one or more substituents selected from -OH, halogen atoms (Cl, Br, I and F), groups -NR4R5, where R4 and R5, which are identical or different, represent hydrogen or a C1 to C6 linear or branched alkyl group or a phenyl group; and/or polyoxyalkylene groups, especially polyoxyethylene and/or polyoxypropylene, said polyoxyalkylene group consisting of the repetition of 5 to 30 oxyalkylene units;
  - a C3 to C12 cycloalkyl group, said cycloalkyl group being able to contain, in its chain, one or more heteroatoms selected from O, N, S and/or P, and/or to be optionally substituted by one or more substituents

selected from -OH and halogen atoms (Cl, Br, I and F);
- a C4 to C20 aryl or C5 to C30 aralkyl group (Cl to C8 alkyl group);

in particular R may be a methyl, ethyl, propyl, isopropyl, n-butyl, isobutyl, tert-butyl, hexyl, ethylhexyl, octyl, lauryl, isooctyl, isodecyl, dodecyl, cyclohexyl, t-butylcyclohexyl, stearyl, 2-ethylperfluorohexyl, 2-hydroxyethyl, 2-hydroxybutyl, 2-hydroxypropyl, methoxyethyl, ethoxyethyl, methoxypropyl, isobornyl, phenyl, 2-phenylethyl, t-butylbenzyl, benzyl, furfurylmethyl or tetrahydrofurfurylmethyl group, a methoxypolyoxyethylene (or POE-methyl) group; a POE-behenyl or trifluoroethyl group; or a dimethylaminoethyl, diethylaminoethyl or dimethylaminopropyl group;
- (ii) (meth)acrylamides of formula $CH_2$=CHCONR4R5 or $CH_2$=C($CH_3$)CONR4R5
in which R4 and R5, which are identical or different, represent a hydrogen atom or:

a) a linear or branched alkyl group containing 1 to 18 carbon atoms, in which there is or are optionally intercalated one or more heteroatoms selected from O, N, S and P; it being possible additionally for said alkyl group to be optionally substituted by one or more substituents selected from hydroxyl groups, halogen atoms (Cl, Br, I and F) and groups Si(R4R5), in which R4 and R5, which are identical or different, represent a C1 to C6 alkyl group or a phenyl group;
and in particular a methyl, ethyl, propyl, isopropyl, n-butyl, isobutyl, tert-butyl, hexyl, isohexyl, cyclohexyl, ethylhexyl, octyl, isooctyl, decyl, isodecyl, cyclodecyl, dodecyl, cyclododecyl, isononyl, lauryl, t-butylcyclohexyl or stearyl group; a 2-ethylperfluorohexyl group; or a C1-4 hydroxyalkyl group such as 2-hydroxyethyl, 2-hydroxybutyl and 2-hydroxypropyl; or a (C1-4)alkoxy-(C1-4) alkyl group such as methoxyethyl, ethoxyethyl and methoxypropyl,
b) a C3 to C12 cycloalkyl group, such as the isobornyl group, or a heterocycloalkyl group (with alkyl of 1 to 4 carbon atoms), such as furfurylmethyl or tetrahydrofurfurylmethyl,
c) a C4 to C20 aryl group such as the phenyl group, or
d) a C5 to C30 aralkyl group (with a C1 to C8 alkyl group) such as 2-phenylethyl, t-butylbenzyl or benzyl;

- (iii) ethylenically unsaturated monomers containing at least one carboxylic, phosphoric or sulphonic acid or anhydride function, such as, for example, acrylic acid, methacrylic acid, crotonic acid, maleic anhydride, itaconic acid, fumaric acid, maleic acid, styrenesulphonic acid, vinylbenzoic acid, vinylphosphoric acid or acrylamidopropanesulphonic acid; and the salts thereof;
- (iv) vinyl ethers of formula $R_6$O-CH=$CH_2$ or vinyl esters of formula $R_6$-COO-CH=$CH_2$
in which $R_6$ represents a linear or branched alkyl group containing 1 to 22 carbon atoms or a cyclic alkyl group containing 3 to 6 carbon atoms and/or an aromatic group, for example of benzene, anthracene or naphthalene type;
- (v) vinyl compounds of formulae $CH_2$=CH-R9, $CH_2$=CH-$CH_2$-R9 or $CH_2$=C($CH_3$)=$CH_2$-R9
in which R9 is a hydroxyl, halogen (Cl or F) or $NH_2$ group or a group OR10 in which R10 represents a phenyl group or a C1 to C12 alkyl group (the monomer is a vinyl or allyl ether); an acetamide group ($NHCOCH_3$); a group OCOR11 in which R11 represents an alkyl group of 2 to 12 carbons which is linear or branched (the monomer is a vinyl or allyl ester); or a group selected from:

- a linear or branched alkyl group of 1 to 18 carbon atoms, in which there is or are optionally intercalated one or more heteroatoms selected from O, N, S and P, it being possible for said alkyl group additionally to be optionally substituted by one or more substituents selected from hydroxyl groups, halogen atoms (Cl, Br, I and F) and groups Si(R4R5), in which R4 and R5, which are identical or different, represent a C1 to C6 alkyl group or a phenyl group;
- a C3 to C12 cycloalkyl group such as isobornyl or cyclohexane,
- a C3 to C20 aryl group such as phenyl,
- a C4 to C30 aralkyl group (with a C1 to C8 alkyl group) such as 2-phenylethyl or benzyl,
- a 4- to 12-membered heterocyclic group containing one or more heteroatoms selected from O, N and S, the ring being aromatic or nonaromatic, and
- a heterocycloalkyl group (with an alkyl of 1 to 4 C), such as furfurylmethyl or tetrahydrofurfurylmethyl;

- (vi) styrene and its derivatives, particularly such as methylstyrene, chlorostyrene or chloromethylstyrene;
- (vii) ethylenically unsaturated monomers containing one or more silicon atoms, such as methacryloyloxypropyltrimethoxysiloxane and methacryloyloxypropyltris(trimethylsiloxy)silane;
- and also their salts, and mixtures thereof.

**15.** Copolymer according to either of Claims 13 and 14, wherein the additional monomer is selected from methyl, ethyl, n-propyl, isopropyl, n-butyl, t-butyl, cyclohexyl, methoxyethyl, ethoxyethyl, trifluoroethyl, dimethylaminoethyl, diethylaminoethyl, 2-hydroxypropyl and 2-hydroxyethyl (meth)acrylates; acrylic acid, methacrylic acid, (meth)acrylamide, methacryloyloxypropyltrimethoxysilane, methacryloyloxypropyltris(trimethylsiloxy)silane; and also their salts; and mixtures thereof.

**16.** Copolymer according to one of Claims 13 to 15, wherein the additional monomer is selected from carbon or silicone macromonomers having at least one polymerizable end group, especially a vinyl or (meth)acrylate group.

**17.** Copolymer according to Claim 16, wherein the additional macromonomer is selected, alone or in a mixture, from the following, and also their salts:

- (i) linear or branched C8-C22 alkyl (meth)acrylate homopolymers and copolymers having a polymerizable end group selected from vinyl or (meth)acrylate groups, among which mention may be made of poly(2-ethylhexyl acrylate) macromonomers having a mono(meth)acrylate end group; poly(dodecyl acrylate) or poly(dodecyl methacrylate) macromonomers having a mono(meth)acrylate end group; and poly(stearyl acrylate) or poly (stearyl methacrylate) macromonomers having a mono(meth)acrylate end group,
- (ii) polyolefins having an ethylenically unsaturated end group, especially those having a (meth)acrylate end group; and in particular the following macromonomers, on the understanding that they have a (meth)acrylate end group: polyethylene macromonomers, polypropylene macromonomers, polyethylene/polypropylene copolymer macromonomers, polyethylene/polybutylene copolymer macromonomers, polyisobutylene macromonomers; polybutadiene macromonomers; polyisoprene macromonomers; and poly(ethylene/butylene)-polyisoprene macromonomers,
- (iii) polydimethylsiloxanes having a mono (meth)-acrylate end group, and especially those of formula (IIa) below:

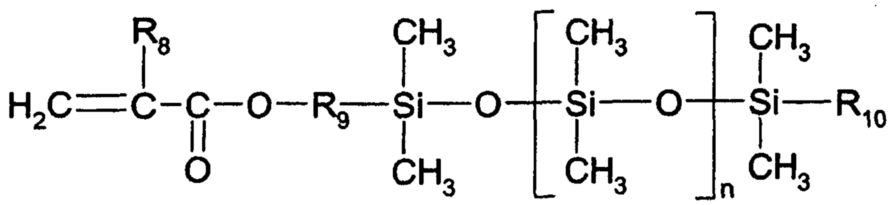

(IIa)

in which:

- $R_8$ denotes a hydrogen atom or a methyl group; preferably a methyl group;
- $R_9$ denotes a linear or branched, preferably linear, divalent hydrocarbon group having 1 to 10 carbon atoms and optionally containing one or two ether bonds -O-; preferably ethylene, propylene or butylene;
- $R_{10}$ denotes a linear or branched alkyl group having 1 to 10 carbon atoms, in particular 2 to 8 carbon atoms; preferably methyl, ethyl, propyl, butyl or pentyl;
- n denotes an integer ranging from 1 to 300, preferably ranging from 3 to 200 and preferentially ranging from 5 to 100.

**18.** Copolymer according to one of the preceding claims, comprising at least one "high-Tg" branch or polymeric sequence, i.e. a branch or sequence having a Tg greater than or equal to 20°C, especially between 20°C and 150°C inclusive, particularly between 30 and 120°C, more particularly between 40°C and 100°C.

**19.** Copolymer according to Claim 18, wherein the high-Tg branch is present in an amount greater than or equal to 50%, especially from 55% to 95%, preferentially from 60% to 90% by weight, relative to the total weight of the polymer.

**20.** Copolymer according to one of the preceding claims, comprising at least one "low-Tg" branch or sequence, i.e. a branch or sequence having a Tg strictly lower than 20°C, in other words between -150°C and 19°C, in particular between -100°C and 0°C, more particularly between -80°C and -5°C, or even between -70°C and -10°C.

**21.** Copolymer according to Claim 20, wherein the low-Tg branch is present in an amount less than 50%, especially from 5% to 45%, preferentially from 10% to 40% by weight, relative to the total weight of the polymer.

**22.** Copolymer according to either of Claims 18 and 19, wherein the high-Tg branch comprises high-Tg monomers present in a proportion of 30% to 100% by weight, especially 40% to 95% by weight, more preferably from 50% to 90% by weight, relative to the total weight of said branch.

**23.** Copolymer according to either of Claims 20 and 21, wherein the low-Tg branch comprises low-Tg monomers present in a proportion of 30% to 100% by weight, especially 40% to 95% by weight, more preferably from 50% to 90% by weight, relative to the total weight of said branch.

**24.** Copolymer according to one of Claims 18 to 23, wherein the main branch is "high-Tg" and at least one of the secondary branches is "low-Tg".

**25.** Copolymer according to one of the preceding claims, comprising at least one "high-Tg" monomer, i.e. a monomer having a Tg greater than or equal to 20°C, especially between 20 and 150°C, more particularly between 30 and 120°C, and better still between 40 and 100°C, or a mixture of such monomers.

**26.** Copolymer according to Claim 25, wherein the "high-Tg" monomer or monomers is or are present in a proportion of 40% to 100% by weight, especially 50% to 80% by weight, more preferably 55% to 70% by weight, relative to the total weight of monomers present in the copolymer.

**27.** Copolymer according to one of the preceding claims, comprising at least one "low-Tg" monomer, i.e. a monomer having a Tg strictly less than 20°C, especially between -150 and 19°C, more particularly between -100 and 0°C, better still between -80 and -5°C, or even between -70°C and -10°C, or a mixture of such monomers.

**28.** Copolymer according to Claim 27, wherein the low-Tg monomer or monomers is or are present in a proportion of 0 to 60% by weight, especially from 20% to 50% by weight, more preferably from 30% to 45% by weight, relative to the total weight of monomers present in the copolymer.

**29.** Copolymer according to one of the preceding claims, comprising at least one lipophilic monomer, which may be present in a proportion of 40% to 100% by weight, especially 50% to 80% by weight, more preferably 55% to 70% by weight, relative to the total weight of monomers present in the copolymer.

**30.** Copolymer according to one of the preceding claims, comprising at least one hydrophilic monomer, which may be present in a proportion of 0% to 60% by weight, especially 20% to 50% by weight, more preferably 30% to 45% by weight, relative to the total weight of monomers present in the copolymer.

**31.** Copolymer according to one of the preceding claims, wherein the main branch is lipophilic, and more preferably wherein the entirety of the branches, both main and secondary, are lipophilic.

**32.** Copolymer according to one of the preceding claims, having a number-average molecular mass of between 2000 g/mol and 1 500 000 g/mol, especially between 5500 g/mol and 1 000 000 g/mol, and more preferably between 6000 g/mol and 900 000 g/mol.

**33.** Copolymer according to one of the preceding claims, soluble at a concentration of at least 3% by weight in isododecane at 25°C and 1 atm, preferably at a concentration of at least 5% by weight, or even of at least 10% by weight.

**34.** Cosmetic or pharmaceutical composition, especially topical dermatological composition, comprising, in a physiologically acceptable medium, especially a cosmetically or dermatologically acceptable medium, at least one hyperbranched copolymer comprising at least two, especially three, polymeric branches, each of which contains at least one at least trifunctional branch point, different from and independent of one another, wherein a first branch contains at least one first monomer selected from the group consisting of isobornyl acrylate, isobornyl methacrylate, isobutyl acrylate, isobutyl methacrylate and 2-ethylhexyl acrylate, and in that at least one second branch contains at least one second monomer, identical to or different from said first monomer, selected from said group.

**35.** Composition according to Claim 34, wherein the copolymer is present in an amount of 0.1% to 95% by weight, preferably 0.5% to 90% by weight, especially 1% to 80% by weight, or even 5%-70% by weight, relative to the total weight of the composition.

**36.** Composition according to either of Claims 34 and 35, comprising at least one fatty phase, which comprises at least

one constituent selected from oils, solvents, preferably lipophilic solvents, fatty substances which are solid at ambient temperature, such as waxes, pasty fatty substances, gums, and mixtures thereof.

**37.** Composition according to Claim 36, comprising at least one constituent selected from oils and/or solvents having an overall solubility parameter according to the Hansen solubility space of less than or equal to 20 $(MPa)^{1/2}$, preferably less than or equal to 18 $(MPa)^{1/2}$, more preferably less than or equal to 17 $(MPa)^{1/2}$.

**38.** Composition according to Claim 37, wherein the oils and/or solvents having an overall solubility parameter according to the Hansen solubility space of less than or equal to 20 $(MPa)^{1/2}$ are selected from oils, volatile or non-volatile, which may be selected from natural or synthetic, carbon, hydrocarbon and fluoro oils, optionally branched, alone or in a mixture; ethers and esters having more than 6 carbon atoms, especially 6 to 30 carbon atoms; ketones having more than 6 carbon atoms, especially 6 to 30 carbon atoms; and aliphatic fatty monoalcohols having 6 to 30 carbon atoms, the hydrocarbon chain containing no substituent group.

**39.** Composition according to Claim 38, wherein the oils and/or solvents are selected from non-volatile carbon oils, especially hydrocarbon oils, of plant, mineral, animal or synthetic origin, such as liquid paraffin (or vaseline), squalane, hydrogenated polyisobutylene (Parleam oil), perhydrosqualene, mink oil, macadamia oil, turtle oil, soya oil, sweet almond oil, calophyllum oil, palm oil, grapeseed oil, sesame oil, corn oil, arara oil, rapeseed oil, sunflower oil, cottonseed oil, apricot oil, castor oil, avocado oil, jojoba oil, olive oil or cereal germ oil, or karite butter; linear, branched or cyclic esters having more than 6 carbon atoms, especially 6 to 30 carbon atoms, such as esters of lanolic acid, of oleic acid, of lauric acid and of stearic acid; esters derived from long-chain alcohols or acids (i.e. those having 6 to 20 carbon atoms), especially the esters of formula RCOOR' in which R represents the residue of a higher fatty acid containing 7 to 19 carbon atoms and R' represents a hydrocarbon chain containing 3 to 20 carbon atoms, in particular C12-C36 esters, such as isopropyl myristate, isopropyl palmitate, butyl stearate, hexyl laurate, diisopropyl adipate, isononyl isononanoate, 2-ethylhexyl palmitate, 2-hexyldecyl laurate, 2-octyldecyl palmitate, 2-octyldodecyl myristate or lactate, di(2-ethylhexyl) succinate, diisostearyl malate, glycerol triisostearate or diglycerol triisostearate; higher fatty acids, especially C14-C22 acids, such as myristic acid, palmitic acid, stearic acid, behenic acid, oleic acid, linoleic acid, linolenic acid or isostearic acid; higher fatty alcohols, especially C16-C22 alcohols, such as cetanol, oleyl alcohol, linoleyl alcohol or linolenyl alcohol, isostearyl alcohol or octyldodecanol; and mixtures thereof; decanol, dodecanol, octadecanol, the liquid triglycerides of fatty acids of 4 to 10 carbon atoms, such as the triglycerides of heptanoic or octanoic acid, the triglycerides of caprylic/capric acids; linear or branched hydrocarbons of mineral or synthetic origin, such as liquid paraffins and derivatives thereof, vaseline, polydecenes, hydrogenated polyisobutene such as Parleam; synthetic esters and ethers, particularly those of fatty acids, such as, for example, Purcellin oil, isopropyl myristate, 2-ethylhexyl palmitate, 2-octyldodecyl stearate, 2-octyldodecyl erucate, isostearyl isostearate; hydroxy esters such as isostearyl lactate, octyl hydroxystearate, octyldodecyl hydroxystearate, diisostearyl malate, triisocetyl citrate, and heptanoates, octanoates and decanoates of fatty alcohols; polyol esters such as propylene glycol dioctanoate, neopentyl glycol diheptanoate, diethylene glycol diisononanoate; and esters of pentaerythritol; fatty alcohols having 12 to 26 carbon atoms such as octyldodecanol, 2-butyloctanol, 2-hexyldecanol and 2-undecylpentadecanol; ketones which are liquid at ambient temperature, such as methyl ethyl ketone, methyl isobutyl ketone, diisobutyl ketone, isophorone, cyclohexanone and acetone; propylene glycol ethers which are liquid at ambient temperature, such as propylene glycol monomethyl ether, propylene glycol monomethyl ether acetate, and dipropylene glycol mono-n-butyl ether; short-chain esters (having 3 to 8 carbon atoms in total) such as ethyl acetate, methyl acetate, propyl acetate, n-butyl acetate, and isopentyl acetate; ethers which are liquid at ambient temperature, such as diethyl ether, dimethyl ether or dichlorodiethyl ether; alkanes which are liquid at ambient temperature, such as decane, heptane, dodecane, isododecane, isohexadecane and cyclohexane; cyclic aromatic compounds which are liquid at ambient temperature, such as toluene and xylene; aldehydes which are liquid at ambient temperature, such as benzaldehyde and acetaldehyde, and mixtures thereof; volatile non-silicone oils, especially C8-C16 isoparaffins such as isododecane, isodecane and isohexadecane.

**40.** Composition according to one of Claims 34 to 39, comprising at least one oil and/or solvent selected from volatile or non-volatile alkanes which are liquid at ambient temperature, and more particularly decane, heptane, dodecane, isododecane, isohexadecane, cyclohexane, isodecane, and mixtures thereof.

**41.** Composition according to one of Claims 34 to 40, comprising at least from 0.01% to 95%, preferably from 0.1% to 90%, more preferably from 10% to 85% by weight, relative to the total weight of the composition, and better still from 30% to 80%, of at least one oil and/or solvent.

**42.** Composition according to one of Claims 34 to 41, further comprising a constituent selected from a hydrophilic

medium comprising water or a mixture of water and hydrophilic organic solvent; waxes, gums; colorants; fillers; polymers, especially film-forming polymers; vitamins, thickeners, gelling agents, trace elements, softeners, sequestrants, perfumes, alkalifying or acidifying agents, preservatives, sunscreens, surfactants, antioxidants, anti-hair-loss agents, anti-dandruff agents, propellants, ceramides, or mixtures thereof.

**43.** Composition according to one of Claims 34 to 42, in the form of a suspension, a dispersion, a solution, especially an organic solution, a gel, an emulsion, especially an oil-in-water (O/W) or water-in-oil (W/O) emulsion or multiple (W/O/W or polyol/O/W or O/W/O) emulsion, or the form of a cream, a paste, a mousse, a vesicle dispersion, particularly of ionic or nonionic lipids, a two-phase or multi-phase lotion, a spray, a powder, a paste, especially a flexible paste; or in anhydrous form, for example an anhydrous paste.

**44.** Composition according to one of Claims 34 to 43, in the form of a makeup composition, especially a complexion product such as a foundation, blusher or eyeshadow; a lip product such as a lipstick or a lip care product; a concealer product; a blusher, mascara or eyeliner; an eyebrow makeup product, a lip pencil or eye pencil; a product for the nails such as a nail varnish or nail care product; a body makeup product; a hair makeup product (mascara or lacquer for hair); or in the form of a composition for protecting or caring for the skin of the face, neck, hands or body, especially an anti-wrinkle composition or anti-fatigue composition allowing a glow to be imparted to the skin, or a moisturizing or treatment composition; or an anti-sun or artificial tanning composition; or a hair composition, especially for holding the hairstyle or shaping the hair.

**45.** Composition according to Claim 44, in the form of a makeup composition, especially a foundation or a lipstick.

**46.** Cosmetic method of making up or caring for keratin materials, especially the skin of the body or the face, the lips, the nails, the hair and/or the eyelashes, comprising applying to said materials a cosmetic composition as defined in one of Claims 34 to 45.

**47.** Cosmetic method of making up the skin of the face and/or the lips, comprising applying to said materials a cosmetic foundation or lipstick composition as defined in Claim 46.

## Patentansprüche

**1.** Hochverzweigtes Copolymer, das mindestens zwei und insbesondere drei Polymerzweige aufweist, die jeder mindestens einen mindestens dreiwertigen Verzweigungspunkt aufweisen, der jeweils von dem anderen verschieden und unabhängig ist, bei dem

- ein erster Zweig mindestens ein erstes Monomer aufweist, das unter Isobornylacrylat und Isobornylmethacrylat ausgewählt ist, und
- mindestens ein zweiter Zweig mindestens ein zweites Monomer enthält, das mit dem ersten Monomer identisch oder davon verschieden ist und unter Isobornylacrylat, Isobornylmethacrylat, Isobutylacrylat, Isobutylmethacrylat und 2-Ethylhexylacrylat ausgewählt ist.

**2.** Copolymer nach Anspruch 1, wobei die Zweige über eine multifunktionelle Verbindung miteinander verbunden sind, die mindestens zwei polymerisierbare funktionelle Gruppen aufweist.

**3.** Copolymer nach Anspruch 2, wobei die multifunktionelle Verbindung der folgenden Formel (I) entspricht:

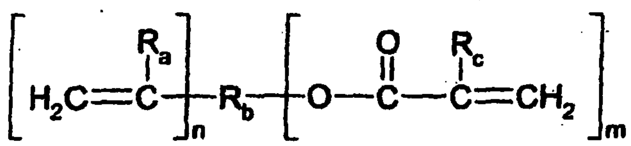

(I)

wobei in der Formel:

- Ra und Rc unabhängig von einander unter Wasserstoff oder den geradkettigen oder verzweigten Alkylgruppen mit 1 bis 22 Kohlenstoffatomen und insbesondere 1 bis 3 Kohlenstoffatomen ausgewählt sind; vorzugsweise kann Ra ein Wasserstoffatom bedeuten; vorzugsweise kann Rc die Methylgruppe bedeuten;
- Rb ist eine geradkettige oder verzweigte Alkylengruppe mit 1 bis 22 Kohlenstoffatomen oder eine Cycloalkylengruppe mit 3 bis 6 Kohlenstoffatomen oder eine Arylengruppe mit 6 bis 18 Kohlenstoffatomen oder eine Alkarylengruppe mit 7 bis 24 Kohlenstoffatomen; vorzugsweise bedeutet Rb die Methylengruppe;
- m und n bedeuten unabhängig voneinander 1, 2, 3 oder 4,

wobei sie so gewählt sind, dass die Summe m+n größer oder gleich 2 ist; vorzugsweise m=n=1.

**4.** Copolymer nach Anspruch 3, wobei die multifunktionelle Verbindung ein Allyl(meth)acrylat und besser das Allylmethacrylat ist.

**5.** Copolymer nach einem der vorhergehenden Ansprüche, wobei jeder Zweig in Form einer Polymersequenz vom Typ eines Homopolymers oder vom Typ eines statistischen Copolymers, alternierenden Copolymers, Blockcopolymers oder Gradientencopolymers vorliegt.

**6.** Copolymer nach einem der vorhergehenden Ansprüche, wobei jeder Zweig in Form eines Homopolymers oder statistischen linearen Copolymers vorliegt.

**7.** Copolymer nach einem der vorhergehenden Ansprüche, wobei der Hauptzweig in Form eines Homopolymers oder statistischen Copolymers vorliegt, der, bezogen auf das Gewicht des Zweigs, 80 bis 100 Gew.-% und vorzugsweise 100 Gew.-% Monomer(e) enthält, das (die) unter Isobornylacrylat, Isobornylmethacrylat, Isobutylacrylat, Isobutylmethacrylat und 2-Ethylhexylacrylat ausgewählt ist (sind).

**8.** Copolymer nach einem der vorhergehenden Ansprüche, wobei der/die Nebenzweig(e) in Form eines Homopolymers oder statistischen Copolymers vorliegt (vorliegen), das, bezogen auf das Gewicht des Zweigs, 80-100 Gew.-% und vorzugsweise 100 Gew.-% Monomer(e) enthält, das (die) unter Isobornylacrylat, Isobornylmethacrylat, Isobutylacrylat, Isobutylmethacrylat und 2-Ethylhexylacrylat ausgewählt ist (sind).

**9.** Copolymer nach einem der vorhergehenden Ansprüche, bei dem das Gesamtgewicht des Monomers (der Monomere), das (die) unter Isobornylacrylat, Isobornylmethacrylat, Isobutylacrylat, Isobutylmethacrylat und 2-Ethylhexylacrylat ausgewählt ist (sind), in dem fertigen Copolymer im Bereich von 50 bis 100 Gew.-%, insbesondere 60 bis 98 Gew.-%, sogar 70 bis 97 Gew.-%, noch besser 80 bis 96 Gew.-% und bevorzugt 90 bis 95 Gew.-%, bezogen auf das Gesamtgewicht der in dem fertigen Copolymer enthaltenen Monomere, liegt.

**10.** Copolymer nach einem der vorhergehenden Ansprüche, das optional, bezogen auf das Gesamtgewicht des fertigen Copolymers, 1 bis 40 Gew.-%, insbesondere 5 bis 30 Gew.-%, und sogar 10 bis 25 Gew.-% eines dritten Monomers enthält, das unter Isobornylacrylat, Isobornylmethacrylat, Isobutylacrylat, Isobutylmethacrylat und 2-Ethylhexylacrylat ausgewählt ist, wobei die prozentualen Mengenanteile bezogen auf das Gesamtgewicht der in dem fertigen Copolymer enthaltenen Monomere angegeben sind.

**11.** Copolymer nach einem der vorhergehenden Ansprüche, das in dem gleichen Zweig oder in unterschiedlichen Zweigen Kombinationen der folgenden Monomere enthält:

- Isobornylacrylat und Isobornylmethacrylat;
- Isobornylacrylat und Isobutylacrylat;
- Isobornylacrylat und Isobutylmethacrylat;
- Isobornylacrylat und 2-Ethylhexylacrylat;
- Isobornylmethacrylat und Isobutylacrylat;
- Isobornylmethacrylat und Isobutylmethacrylat;
- Isobornylmethacrylat und 2-Ethylhexylacrylat;
- Isobornylacrylat, Isobornylmethacrylat und 2-Ethylhexylacrylat;
- Isobornylacrylat, Isobornylmethacrylat und Isobutylacrylat;
- Isobornylacrylat, Isobutylacrylat und 2-Ethylhexylacrylat;
- Isobornylacrylat, Isobutylmethacrylat und Isobutylacrylat; und
- Isobornylmethacrylat, Isobutylmethacrylat und Isobutylacrylat.

**12.** Copolymer nach einem der vorhergehenden Ansprüche, unfassend:

- Isobornylacrylat in den Hauptzweigen und Isobutylacrylat in den Nebenzweigen;
- Isobornylacrylat in den Hauptzweigen und 2-Ethylhexylacrylat in den Nebenzweigen;
- Isobornylmethacrylat in den Hauptzweigen und Isobutylacrylat in den Nebenzweigen;
- Isobornylmethacrylat in den Hauptzweigen und 2-Ethylhexylacrylat in den Nebenzweigen;
- Isobornylacrylat und Isobornylmethacrylat in den Hauptzweigen und Isobutylacrylat in den Nebenzweigen;
- Isobornylacrylat und Isobornylmethacrylat in den Hauptzweigen und 2-Ethylhexylacrylat in den Nebenzweigen;
- Isobornylacrylat und Isobutylmethacrylat in den Hauptzweigen und Isobutylacrylat in den Nebenzweigen;
- Isobornylacrylat und Isobutylmethacrylat in den Hauptzweigen und 2-Ethylhexylacrylat in den Nebenzweigen;
- Isobornylmethacrylat und Isobutylmethacrylat in den Hauptzweigen und Isobutylacrylat in den Nebenzweigen; oder
- Isobornylmethacrylat und Isobutylmethacrylat in den Hauptzweigen und 2-Ethylhexylacrylat in den Nebenzweigen.

**13.** Copolymer nach einem der vorhergehenden Ansprüche, das ferner mindestens ein ergänzendes Monomer enthält, das in einen Mengenanteil von 0 bis 50 Gew.-%, insbesondere 2 bis 40 Gew.-%, sogar 3 bis 30 Gew.-%, noch besser 4 bis 20 Gew.-% und vorzugsweise 5 bis 10 Gew.-%, bezogen auf das Gesamtgewicht der in dem fertigen Copolymer vorhandenen Monomere, enthalten sein kann.

**14.** Copolymer nach Anspruch 13, wobei das ergänzende Monomer einzeln oder in Form eines Gemisches unter den folgenden Monomeren sowie deren Salzen ausgewählt ist:

- (i) (Meth)acrylaten der Formel $CH_2$=CHCOOR oder $CH_2$=C($CH_3$)COOR, worin R bedeutet:

  - eine geradkettige oder verzweigte Alkylgruppe mit 1 bis 30 Kohlenstoffatomen, bei der sich gegebenenfalls in der Kette ein oder mehrere Heteroatome befindet (befinden), die unter O, N, S und P ausgewählt sind, und/oder wobei die Alkylgruppe gegebenenfalls mit einem oder mehreren Substituenten substituiert sein kann, die ausgewählt sind unter -OH, Halogenatomen (Cl, Br, I und F), Gruppen -$NR_4R_5$, wobei $R_4$ und $R_5$, die gleich oder verschieden sind, Wasserstoff oder eine geradkettige oder verzweigte $C_{1-6}$-Alkylgruppe oder eine Phenylgruppe bedeuten; und/oder Polyoxyalkylengruppen, insbesondere Polyoxyethylen und/oder Polyoxypropylen, wobei die Polyoxyalkylengruppe aus Wiederholungen von 5 bis 30 Oxyalkyleneinheiten besteht;
  - eine Cycloalkylgruppe mit 3 bis 12 Kohlenstoffatomen, wobei die Cycloalkylgruppe in ihrer Kette ein oder mehrere Heteroatome enthalten kann, die unter O, N, S und/oder P ausgewählt sind, und/oder wobei sie gegebenenfalls mit einem oder mehreren Substituenten substituiert sein kann, die unter -OH und den Halogenatomen (Cl, Br, I und F) ausgewählt sind;
  - eine Arylgruppe mit 4 bis 20 Kohlenstoffatomen oder eine Aralkylgruppe mit 5 bis 30 Kohlenstoffatomen ($C_{1-8}$-Alkylgruppe),

  insbesondere kann R eine der folgenden Gruppen sein: Methyl, Ethyl, Propyl, Isopropyl, *n*-Butyl, Isobutyl, *tert*-Butyl, Hexyl, Ethylhexyl, Octyl, Lauryl, Isooctyl, Isodecyl, Dodecyl, Cyclohexyl, *tert*-Butylcyclohexyl, Stearyl, 2-Ethylperfluorhexyl, 2-Hydroxyethyl, 2-Hydroxybutyl, 2-Hydroxypropyl, Methoxyethyl, Ethoxyethyl, Methoxypropyl, Isobornyl, Phenyl, 2-Phenylethyl, *tert*-Butylbenzyl, Benzyl, Furfurylmethyl oder Tetrahydrofurylmethyl, Methoxypolyoxyethylen (oder POE-Methyl); POE-Behenyl, Trifluorethyl; Dimethylaminomethyl; Diethylaminoethyl, Dimethylaminopropyl;

- (ii) (Meth)acrylamiden der Formel $CH_2$=$CHCONR_4R_5$ oder $CH_2$=C($CH_3$)$CONR_4R_5$,
wobei die Gruppen $R_4$ und $R_5$, die gleich oder verschieden sind, ein Wasserstoffatom bedeuten oder:

  a) eine geradkettige oder verzweigte Alkylgruppe mit 1 bis 18 Kohlenstoffatomen, bei der sich gegebenenfalls in der Kette ein oder mehrere Heteroatome befindet (befinden), die unter O, N, S und P ausgewählt sind; wobei die Alkylgruppe ferner gegebenenfalls mit einem oder mehreren Substituenten substituiert sein kann, die unter den Hydroxygruppen, Halogenatomen (Cl, Br, I und F) und den Gruppen Si($R_4R_5$) ausgewählt sind, wobei $R_4$ und $R_5$, die gleich oder verschieden sind, eine $C_{1-6}$-Alkylgruppe oder eine Phenylgruppe bedeuten;
  und insbesondere eine der folgenden Gruppen sein: Methyl, Ethyl, Propyl, Isopropyl, *n*-Butyl, Isobutyl, *tert*-Butyl, Hexyl, Isohexyl, Cyclohexyl, Ethylhexyl, Octyl, Isooctyl, Decyl, Isodecyl, Cyclodecyl, Dodecyl, Cyclododecyl, Isononyl, Lauryl, *tert*-Butylcyclohexyl, Stearyl; 2-Ethylperfluorhexyl; oder eine Hydroxyalkyl

($C_{1-4}$)gruppe, wie 2-Hydroxyethyl, 2-Hydroxybutyl und 2-Hydroxypropyl; oder eine Alkoxy($C_{1-4}$)alkyl($C_{1-4}$) gruppe, wie Methoxyethyl, Ethoxyethyl und Methoxypropyl,

b) eine Cycloalkylgruppe mit 3 bis 12 Kohlenstoffatomen, wie Isobornyl, oder eine Heterocycloalkylgruppe (Alkyl mit 1 bis 4 Kohlenstoffatomen, wie Furfurylmethyl oder Tetrahydrofurfurylmethyl,

c) eine Arylgruppe mit 4 bis 20 Kohlenstoffatomen, wie Phenyl,

d) eine Aralkylgruppe mit 5 bis 30 Kohlenstoffatomen (Al-kylgruppe mit 1- 8 C), wie 2-Phenylethyl, *tert*-Butylbenzyl oder Benzyl,

- (iii) Monomeren mit einer oder mehreren ethylenisch ungesättigten Bindungen, die mindestens eine Carbonsäurefunktion, Phosphorsäurefunktion oder Sulfonsäurefunktion oder Anhydridfunktion aufweisen, wie beispielsweise Acrylsäure, Methacylsäure, Crotonsäure, Maleinsäureanhydrid, Itaconsäure, Fumarsäure, Maleinsäure, Styrolsulfonsäure, Vinylbenzoesäure, Vinylphosphorsäure, Acrylamidopropansulfonsäure; und den Salzen dieser Verbindungen,
- (iv) Vinylethern der Formel $R_6O\text{-}CH=CH_2$ oder Vinylestern der Formel: $R_6\text{-}COO\text{-}CH=CH_2$, wobei $R_6$ eine geradkettige oder verzweigte Alkylgruppe mit 1 bis 22 Kohlenstoffatomen bedeutet oder eine cyclische Alkylgruppe mit 3 bis 6 Kohlenstoffatomen und/oder eine aromatische Gruppe, beispielsweise vom Benzoltyp, Anthracentyp und Naphthalintyp;
- (v) Vinylverbindungen der Formeln: $CH_2=CH\text{-}R_9$, $CH_2=CH\text{-}CH_2\text{-}R_9$ oder $CH_2=C(CH_3)\text{-}CH_2R_9$, worin $R_9$ eine Hydroxygruppe, Halogen (C1 oder F), $NH_2$, $OR_{10}$, wobei $R_{10}$ eine Phenylgruppe oder $C_{1-12}$-Alkylgruppe bedeutet (das Monomer ist ein Vinylether oder Allylether); Acetamid ($NHCOCH_3$); eine Gruppe $OCOR_{11}$, worin $R_{11}$ eine geradkettige oder verzweigte Alkylgruppe mit 2 bis 12 Kohlenstoffatomen ist (das Monomer ist ein Vinylester oder Allylester); oder eine Gruppe, die ausgewählt ist unter:

  - einer geradkettigen oder verzweigten Alkylgruppe mit 1 bis 18 Kohlenstoffatomen, worin gegebenenfalls in der Kette ein oder mehrere Heteroatome enthalten ist (sind), die unter O, N, S und P ausgewählt sind; wobei die Alkylgruppe ferner gegebenenfalls mit einem oder mehreren Substituenten substituiert sein kann, die unter den Hydroxygruppen, Halogenatomen (Cl, Br, I und F) und Gruppen $Si(R_4R_5)$ ausgewählt sind, wobei $R_4$ und $R_5$, die gleich oder verschieden sind, eine $C_{1-6}$-Alkylgruppe oder eine Phenylgruppe bedeuten;
  - einer Cycloalkylgruppe mit 3 bis 12 Kohlenstoffatomen, wie Isobornyl, Cyclohexan,
  - einer Arylgruppe mit 3 bis 20 Kohlenstoffatomen, wie Phenyl,
  - einer Aralkylgruppe mit 4 bis 30 Kohlenstoffatomen (Alkylgruppe mit 1 - 8 C), wie 2-Phenylethyl; Benzyl,
  - einer heterocyclischen Gruppe mit 4 bis 12 Bestandteilen, die ein oder mehrere Heteroatome enthält, die unter O, N und S ausgewählt sind, wobei der Ring aromatisch oder nichtaromatisch sein kann,
  - einer Heterocycloalkylgruppe ($C_{1-4}$-Alkyl), wie Furfurylmethyl oder Tetrahydrofurfurylmethyl,

- (vi) Styrol und seinen Derivaten, insbesondere Methylstyrol, Chlorstyrol oder Chlormethylstyrol;
- (vii) Monomeren mit ethylenisch ungesättigter Bindung, die ein oder mehrere Siliciumatome enthalten, wie Methacryloxypropyltrimethoxysilan, Methacryloxypropyltris(trimethylsiloxy)silan;
- sowie ihren Salzen und ihren Gemischen.

**15.** Copolymer nach einem der Ansprüche 13 bis 14, wobei das ergänzende Monomer ausgewählt ist unter Methyl (meth)acrylat, Ethyl(meth)acrylat, *n*-Propyl(meth)acrylat, Isopropyl(meth)acrylat, *n*-Butyl(meth)acrylat, *tert*-Butyl (meth)acrylat, Cyclohexyl(meth)-acrylat, Methoxyethyl(meth)acrylat, Ethoxyethyl(meth)acrylat, Trifluorethyl(meth) acrylat, Dimethylaminoethyl(meth)acrylat, Diethylaminoethyl(meth)acrylat, 2-Hydroxypropyl(meth)acrylat, 2-Ethoxyethyl(meth)acrylat,; Acrylsäure, Methacrylsäure, (Meth)acrylamid, Methacryloxypropyltrimethoxysilan, Methacryloxypropyltris(trimethylsiloxy)silan; sowie ihren Salzen; und ihren Gemischen.

**16.** Copolymer nach einem der Ansprüche 13 bis 15, wobei das ergänzende Monomer unter den Kohlenstoffmakromonomeren oder Siliconmakromonomeren ausgewählt ist, die mindestens eine polymerisierbare endständige Gruppe aufweisen, insbesondere eine Vinylgruppe oder (Meth)acrylatgruppe.

**17.** Copolymer nach Anspruch 16, wobei das ergänzende Makromonomer einzeln oder in Form von Gemischen unter den folgenden Verbindungen sowie ihren Salzen ausgewählt ist:

- (i) Homopolymeren und Copolymeren von Alkyl(meth)acrylat mit linearer oder verzweigter $C_{8-22}$-Alkylgruppe, die eine endständige polymerisierbare Gruppe aufweisen, die unter Vinyl oder (Meth)acrylat ausgewählt ist, wobei von diesen die Makromonomere von Poly(2-ethylhexylacrylat) mit Mono(meth)acrylat-Endgruppe; Makromonomere von Poly(dodecylacrylat) oder Poly(dodecyl(meth)acrylat) mit Mono(meth)acrylat-Endgruppe;

die Makromonomere von Poly(stearylacrylat) oder Poly(stearyl(meth)acrylat) mit Mono(meth)acryalat-Endgruppe angegeben werden können.

- (ii) Polyolefinen, die eine entständige Gruppe mit ethylenisch ungesättigter Bindung aufweisen, insbesondere solche mit einer endständigen (Meth)acrylatgruppe; und besonders die folgenden Makromonomere, mit der Maßgabe, dass sie eine endständige (Meth)acrylatgruppe aufweisen: Makromonomere von Polyethylen, Makromonomere von Polypropylen, Makromonomere aus Polyethylen/Polypropylen-Copolymer, Makromonomere aus Polyethylen/Polybutylen-Copolymer, Makromonere von Polyisobutylen; Makromonomere von Polybutadien; Makromonomere von Polyisopren; Makromonomere von Polybutadien; Makromonomere von Poly(ethylen/butylen)-polyisopren;

- (iii) Polydimethylsiloxanen mit endständiger Mono(meth)-acrylatgruppe und besonders solchen der folgenden Formel (IIa):

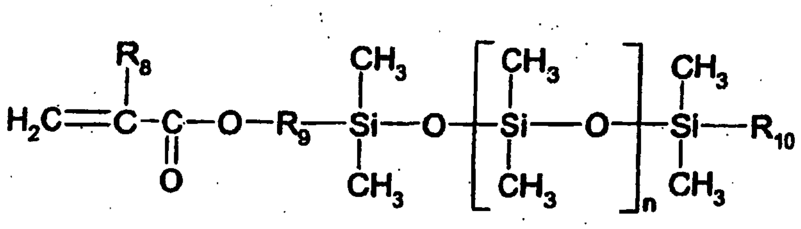

(IIa)

worin bedeuten:

- $R_8$ ein Wasserstoffatom oder die Methylgruppe; vorzugsweise Methyl;
- $R_9$ eine zweiwertige, geradkettige oder verzweigte und vorzugsweise lineare Kohlenwasserstoffgruppe mit 1 bis 10 Kohlenstoffatomen, die gegebenenfalls eine oder zwei Etherbindungen -O- aufweist; vorzugsweise Ethylen, Propylen oder Butylen;
- Rio eine geradkettige oder verzweigte Alkylgruppe mit 1 bis 10 Kohlenstoffatomen, insbesondere 2 bis 8 Kohlenstoffatomen; bevorzugt Methyl, Ethyl, Propyl, Butyl oder Pentyl;
- n eine ganze Zahl von 1 bis 300, vorzugsweise 3 bis 200 und bevorzugt 5 bis 100.

**18.** Copolymer nach einem der vorhergehenden Ansprüche, das mindestens eine Polymersequenz oder einen Zweig aufweist, der als mit "hoher Tg" bezeichnet wird, d. h. mit einer Tg von 20 °C oder darüber, insbesondere im Bereich von 20 bis 150 °C, besonders im Bereich von 30 bis 120 °C und insbesondere im Bereich von 40 bis 100 °C.

**19.** Copolymer nach Anspruch 18, bei dem der Zweig mit hoher Tg in einen Mengenanteil von mindestens 50 %, insbesondere 55 bis 95 %, vorzugsweise 60 bis 90 Gew.-%, bezogen auf das Gesamtgewicht des Polymers, enthalten ist.

**20.** Copolymer nach einem der vorhergehenden Ansprüche, das mindestens eine Sequenz oder einen Zweig aufweist, der als mit "niedriger Tg" bezeichnet wird, d. h. einer Tg immer unter 20 °C, d. h. im Bereich von -150 bis 19 °C, insbesondere im Bereich von -100 bis 0 °C, besonders bevorzugt im Bereich von -80 bis -5 °C und sogar im Bereich von -70 bis -10 °C.

**21.** Copolymer nach Anspruch 20, wobei der Zweig mit niedriger Tg in einen Mengenanteil unter 50 %, insbesondere im Bereich von 5 bis 45 % und vorzugsweise im Bereich von 10 bis 40 Gew.-%, bezogen auf das Gesamtgewicht des Polymers, enthalten ist.

**22.** Copolymer nach einem der Ansprüche 18 bis 19, wobei der Zweig mit hoher Tg Monomere mit hoher Tg enthält, die in einen Mengenanteil von 30 bis 100 Gew.-%, insbesondere 40 bis 95 Gew.-% und besser 50 bis 90 Gew.-%, bezogen auf das Gesamtgewicht des Zweigs, enthalten sind.

**23.** Copolymer nach einem der Ansprüche 20 bis 21, wobei der Zweig mit niedriger Tg Monomere mit niedriger Tg enthält, die in einen Mengenanteil von 30 bis 100 Gew.-%, insbesondere 40 bis 95 Gew.-% und besser 50 bis 90 Gew.-%, bezogen auf das Gesamtgewicht des Zweigs, enthalten sind.

**24.** Copolymer nach einem der Ansprüche 18 bis 23, bei dem der Hauptzweig eine "hohe Tg" aufweist und mindestens

ein Nebenzweig eine "niedrige Tg" hat.

**25.** Copolymer nach einem der vorhergehenden Ansprüche, das mindestens ein Monomer mit einer so genannten "hohen Tg" enthält, d. h. einer Tg von mindestens 20 °C, insbesondere im Bereich von 20 bis 150 °C, besonders im Bereich von 30 bis 120 °C und noch besser im Bereich von 40 bis 100 °C, oder ein Gemisch solcher Monomere.

**26.** Copolymer nach Anspruch 25, wobei das oder die Monomere mit sog. "hoher Tg" in einen Mengenanteil von 40 bis 100 Gew.-%, insbesondere 50 bis 80 Gew.-% und noch besser 55 bis 70 Gew.-%, bezogen auf das Gesamtgewicht der in dem Copolymer enthaltenen Monomere, vorliegen.

**27.** Copolymer nach einem der vorhergehenden Ansprüche, das mindestens ein Monomer mit sog. "niedriger Tg" enthält, d. h. einer Tg immer unter 20 °C, insbesondere im Bereich von -150 bis 19 °C, besonders im Bereich von -100 bis 0 °C, noch besser im Bereich von -80 bis -5 °C und sogar im Bereich von -70 bis -10 °C, oder ein Gemisch solcher Monomere.

**28.** Copolymer nach Anspruch 27, bei dem das/die Monomere mit niedriger Tg in einen Mengenanteil von 0 bis 60 Gew.-%, insbesondere 20 bis 50 Gew.-% und besser 30 bis 45 Gew.-%, bezogen auf das Gesamtgewicht der in dem Copolymer enthaltenen Monomere, enthalten ist (sind).

**29.** Copolymer nach einem der vorhergehenden Ansprüche, das mindestens ein lipophiles Monomer enthält, das in einen Mengenanteil von 40 bis 100 Gew.-%, insbesondere 50 bis 80 Gew.-% und noch besser 55 bis 70 Gew.-%, bezogen auf das Gesamtgewicht der in dem Copolymer enthaltenen Monomere, vorliegen kann.

**30.** Copolymer nach einem der vorhergehenden Ansprüche, das mindestens ein hydrophiles Monomer enthält, das in einen Mengenanteil von 0 bis 60 Gew.-%, insbesondere 20 bis 50 Gew.-% und noch besser 30 bis 45 Gew.-%, bezogen auf das Gesamtgewicht der in dem Copolymer enthaltenen Monomere, enthalten ist.

**31.** Copolymer nach einem der vorhergehenden Ansprüche, bei dem der Hauptzweig lipophil ist und besser, bei dem die gesamten Zweige, der Hauptzweig und die Nebenzweige, lipophil sind.

**32.** Copolymer nach einem der vorhergehenden Ansprüche, das eine zahlenmittlere Molmasse von 2 000 bis 1 500 000 g/mol, insbesondere 5 500 bis 1 000 000 g/mol und noch besser 6 000 bis 900 000 g/mol aufweist.

**33.** Copolymer nach einem der vorhergehenden Ansprüche, das bei 25 °C und 1 atm in einer Konzentration von mindestens 3 Gew.-% in Isododecan löslich ist, vorzugsweise in einer Konzentration von mindestens 5 Gew.-% und besser mindestens 10 Gew.-%.

**34.** Kosmetische oder pharmazeutische Zusammensetzung und insbesondere dermatologische topische Zusammensetzung, die in einem physiologisch akzeptablen Medium und insbesondere einem kosmetisch oder dermatologisch akzeptablen Medium mindestens ein hochverzweigtes Copolymer enthält, das zumindest zwei und insbesondere drei Polymerzweige aufweist, die jeder mindestens einen mindestens dreiwertigen Verzweigungspunkt aufweisen, der jeder von dem anderen verschieden und unabhängig ist,
bei dem ein erster Zweig mindestens ein erstes Monomer enthält, das unter Isobornylacrylat, Isobornylmethacrylat, Isobutylacrylat, Isobutylmethacrylat und 2-Ethylhexylacrylat ausgewählt ist, und mindestens ein zweiter Zweig mindestens ein zweites Monomer enthält, das mit dem ersten Monomer identisch oder davon verschieden ist und aus dieser Gruppe ausgewählt ist.

**35.** Zusammensetzung nach Anspruch 34, wobei das Copolymer in einen Mengenanteil von 0,1 bis 95 Gew.-%, vorzugsweise 0,5 bis 90 Gew.-%, insbesondere 1 bis 80 Gew.-% und sogar 5 bis 70 Gew.-%, bezogen auf das Gesamtgewicht der Zusammensetzung, vorliegt.

**36.** Zusammensetzung nach einem der Ansprüche 34 bis 35, die mindestens eine Fettphase enthält, die mindestens einen Bestandteil aufweist, der unter den Ölen, vorzugsweise lipophilen Lösungsmitteln, bei Umgebungstemperatur festen Fettsubstanzen wie Wachsen, pastösen Fettsubstanzen, Gummis und deren Gemischen ausgewählt ist.

**37.** Zusammensetzung nach Anspruch 36, die mindestens einen Bestandteil enthält, der unter den Ölen und/oder Lösungsmitteln mit einem Gesamtsolubilitätsparameter im Solubilitätsraum nach Hansen von 20 $(MPa)^{1/2}$ oder darunter, vorzugsweise höchstens 18 $(MPa)^{1/2}$ und besser höchstens 17 $(MPa)^{1/2}$ ausgewählt ist.

**38.** Zusammensetzung nach Anspruch 37, wobei die Öle und/oder Lösungsmittel mit einem Gesamtsolubilitätsparameter im Solubilitätsraum nach Hansen von höchstens 20 $(MPa)^{1/2}$ unter den flüchtigen oder nichtflüchtigen Ölen, die unter den natürlichen oder synthetischen Ölen auf Kohlenstoffbasis, Kohlenwasserstoffölen, fluorierten Ölen, die gegebenenfalls verzweigt sind, einzeln oder in Form von Gemischen ausgewählt sein können; Ethern und Estern mit mehr als 6 Kohlenstoffatomen und insbesondere 6 bis 30 Kohlenstoffatomen; Ketonen mit mehr als 6 Kohlenstoffatomen, insbesondere 6 bis 30 Kohlenstoffatomen; aliphatischen Monofettalkoholen mit 6 bis 30 Kohlenstoffatomen, wobei die Kohlenwasserstoffkette keine Substituentengruppe aufweist, ausgewählt sind.

**39.** Zusammensetzung nach Anspruch 38, wobei die Öle und/oder Lösungsmittel ausgewählt sind unter: nichtflüchtigen Ölen auf Kohlenstoffbasis und insbesondere Kohlenwasserstoffölen pflanzlicher, mineralischer, tierischer oder synthetischer Herkunft, wie Paraffinöl (oder Vaseline), Squalan, hydriertem Polyisobutylen (Parleamöl), Perhydrosqualen, Nerzöl, Macadamiaöl, Schildkrötenöl, Sojaöl, Süßmandelöl, Calophyllumöl, Palmöl, Traubenkernöl, Sesamöl, Maisöl, Araraöl, Rapsöl, Sonnenblumenöl, Baumwollsamenöl, Aprikosenkernöl, Ricinusöl, Avocadoöl, Jojobaöl, Olivenöl oder Getreidekeimölen, Sheabutter; linearen, verzweigten oder cyclischen Estern mit mehr als 6 Kohlenstoffatomen, insbesondere 6 bis 30 Kohlenstoffatomen, wie Estern von Lanolinsäure, Ölsäure, Laurinsäure, Stearinsäure; Estern, die von Säuren oder Alkoholen mit langer Kette (d. h. mit 6 bis 20 Kohlenstoffatomen) abgeleitet sind, insbesondere Estern der Formel RCOOR', wobei R den Rest einer höheren Fettsäure mit 7 bis 19 Kohlenstoffatomen und R' eine Kohlenwasserstoffkette mit 3 bis 20 Kohlenstoffatomen bedeutet, insbesondere $C_{12-36}$-Estern, wie Isopropylmyristat, Isopropylpalmitat, Butylstearat, Hexyllaurat, Diisopropyladipat, Isononylisononanoat, 2-Ethylhexylpalmitat, 2-Hexyldecyllaurat, 2-Octyldecylpalmitat, 2-Octyldodecylmyristat, 2-Octyldodecyllactat, Di(2-Ethylhexyl)succinat, Diisostearlymalat, Glyceryltriisostearat oder Diglyceryltriisostearat; höheren Fettsäuren, insbesondere mit 14 bis 22 Kohlenstoffatomen, wie Myristinsäure, Palmitinsäure, Stearinsäure, Behensäure, Ölsäure, Linolsäure, Linolensäure oder Isostearinsäure; höheren Fettalkoholen, insbesondere mit 16 bis 22 Kohlenstoffatomen, wie Cetanol, Oleylalkohol, Linoleylalkohol oder Linolenylalkohol, Isostearylalkohol oder Octyldodecanol; und deren Gemischen; Decanol, Dodecanol, Octadecanol, flüssigen Triglyceriden von Fettsäuren mit 4 bis 10 Kohlenstoffatomen, wie Triglyceriden von Heptansäure oder Octansäure, Triglyceriden von Capryl/ caprinsäure; linearen oder verzweigten Kohlenwasserstoffen mineralischer oder synthetischer Herkunft, wie Paraffinölen und deren Derivaten, Vaseline, Polydecenen, hydriertem Polyisobuten, wie Parleam; synthetischen Estern und Ethern, insbesondere von Fettsäuren, wie beispielsweise Purcellinöl, Isopropylmyristat, 2-Ethylhexylpalmitat, 2-Octyldodecylstearat, 2-Octyldodecylerucat, Isostearylisostearat; hydroxylierten Estern, wie Isostearyllactat, Octylhydroxystearat, Octyldodecylhydroxystearat, Diisostearylmalat, Triisocetylcitrat, Heptanoaten, Octanoaten und Decanoaten von Fettalkoholen; Polyolestern, wie Propylenglycoldioctanoat, Neopentylglycoldiheptanoat, Diethylenglycoldüsononanoat; und Estern von Pentaerythrit; Fettalkoholen mit 12 bis 26 Kohlenstoffatomen, wie Octyldodecanol, 2-Butyloctanol, 2-Hexyldecanol, 2-Undecylpentadecanol; bei Raumtemperatur flüssigen Ketonen, wie Methylethylketon, Methylisobutylketon, Diisobutylketon, Isophoron, Cyclohexanon, Aceton; bei Raumtemperatur flüssigen Propylenglycolethern, wie Propylenglycolmonomethylether, Propylenglycolmonomethyletheracetat, Dipropylenglycolmono-*n*-butylether; kurzkettigen Estern (mit 3 bis 8 Kohlenstoffatomen insgesamt), wie Ethylacetat, Methylacetat, Propylacetat, *n*-Butylacetat, Isopentylacetat; bei Raumtemperatur flüssigen Ethern, wie Diethylether, Dimethylether oder Dichlordiethylether; bei Raumtemperatur flüssigen Alkanen, wie Decan, Heptan, Dodecan, Isododecan, Isohexadecan, Cyclohexan; bei Raumtemperatur flüssigen aromatischen cyclischen Verbindungen, wie Toluol und Xylol; bei Raumtemperatur flüssigen Aldehyden, wie Benzaldehyd, Acetaldehyd und deren Gemischen; nichtsiliconierten flüchtigen Ölen, insbesondere $C_{8-16}$-Isoparaffinen, wie Isododecan, Isodecan und Isohexadecan.

**40.** Zusammensetzung nach einem der Ansprüche 34 bis 39, die mindestens ein Öl und/oder ein Lösungsmittel enthält, das unter den bei Raumtemperatur flüssigen, flüchtigen oder nichtflüchtigen Alkanen und insbesondere Decan, Heptan, Dodecan, Isododecan, Isohexadecan, Cyclohexan, Isodecan und deren Gemischen ausgewählt ist.

**41.** Zusammensetzung nach einem der Ansprüche 34 bis 40, die mindestens 0,01 bis 95 %, vorzugsweise 0,1 bis 90 % und noch bevorzugter 10 bis 85 Gew.-%, bezogen auf das Gesamtgewicht der Zusammensetzung, und besser 30 bis 80 % mindestens eines Öls und/oder Lösungsmittels enthält.

**42.** Zusammensetzung nach einem der Ansprüche 34 bis 41, die ferner einen Bestandteil enthält, der ausgewählt ist unter: einem hydrophilen Medium, das Wasser oder ein Gemisch von Wasser und einem hydrophilen organischen Lösungsmittel enthält; Wachsen, Gummis; Farbmitteln; Füllstoffen; Polymeren und insbesondere filmbildenden Polymeren; Vitaminen, Verdickungsmitteln, Gelbildnern, Spurenelementen, beruhigenden Stoffen, Maskierungsmitteln, Parfums, Alkalisierungsmitteln oder Ansäuerungsmitteln, Konservierungsmitteln, Sonnenschutzfiltern, grenzflächenaktiven Stoffen, Antioxidantien, Wirkstoffen gegen Haarausfall, Antischuppenmitteln, Treibmitteln, Ceramiden oder deren Gemischen.

**43.** Zusammensetzung nach einem der Ansprüche 34 bis 42, die in Form einer Suspension, Dispersion, Lösung und besonders organischen Lösung, als Gel, Emulsion und insbesondere Öl-in-Wasser-Emulsion (O/W) oder Wasser-in-Öl-Emulsion (W/O) oder multiple Emulsion (W/O/W oder Polyol/O/W oder O/W/O), in Form einer Creme, Paste, Mousse, Vesikeldispersion, insbesondere von ionischen oder nichtionischen Lipiden, als zweiphasige oder mehrphasige Lotion, Spray, Pulver, Paste und insbesondere weiche Paste; in wasserfreier Form, beispielsweise als wasserfreie Paste, vorliegt.

**44.** Zusammensetzung nach einem der Ansprüche 34 bis 43, die in Form einer Zusammensetzung zum Schminken vorliegt, insbesondere als Produkt für den Teint, wie als Make-up, Wangenrouge oder Lidschatten; Produkt für die Lippen, wie als Lippenstift oder Lippenpflege; Produkt gegen Augenringe, Blush, Mascara, Eyeliner; Produkt zum Schminken der Augenbrauen, Lippencrayon oder Lidstrichstift; Produkt für die Nägel, wie als Nagellack oder Nagelpflegeprodukt; Produkt zum Schminken des Körpers; Produkt zum Schminken der Haare (Mascara oder Lack für die Haare); Zusammensetzung zum Schutz oder für die Pflege des Gesichts, des Halses, der Hände oder des Körpers und insbesondere als Zusammensetzung gegen Falten und gegen Müdigkeit, das den Teint der Haut zum Strahlen bringen kann, hydratisierende Zusammensetzung oder Zusammensetzung für die Behandlung; Sonnenschutzzusammensetzung oder Zusammensetzung für die künstliche Bräunung; Zusammensetzung für die Haarbehandlung und insbesondere für die Festigung oder Formgebung der Haare vorliegt.

**45.** Zusammensetzung nach Anspruch 44, die in Form einer Zusammensetzung zum Schminken vorliegt, besonders als Make-up oder Lippenstift.

**46.** Kosmetisches Verfahren zum Schminken oder für die Pflege von Keratinsubstanzen und insbesondere der Haut des Körpers oder des Gesichts, der Lippen, der Nägel, der Haare und/oder der Wimpern, das das Auftragen einer kosmetischen Zusammensetzung nach einem der Ansprüche 34 bis 45 auf die Keratinsubstanzen umfasst.

**47.** Kosmetisches Verfahren zum Schminken der Haut des Gesichts und/oder der Lippen, das das Auftragen einer kosmetischen Zusammensetzung für Make-up oder Lippenstift auf die Keratinsubstanzen umfasst, wie in Anspruch 46 definiert.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 1428493 A
- WO 0196429 A
- EP 815848 A
- WO 9735541 A
- EP 895467 A
- EP 96459 A
- US 5625005 A
- WO 9718247 A
- WO 9903894 A

### Littérature non-brevet citée dans la description

- Polymer Handbook. John Wiley, 1989
- **JHO JY ; YOO SH.** *Macromolecules,* 2002, vol. 35, 1146-48
- *Polym. Prepr.,* 1996, vol. 37 (2), 413-14
- **MATYJASZEWSKI.** *Progress in polymer science,* 2001, vol. 26 (3), 337-77
- *Chem review,* 2001, vol. 101 (12), 3737
- **MUELLER.** *Macromolecules,* 2001, vol. 34 (18), 62026-13
- **MATYJASEZWSKI et al.** *JACS,* 1995, vol. 117, 5614
- *Chem review,* 2001, vol. 101 (12), 3681
- *Macromolecules,* 1997, vol. 30, 4238-42
- Macromolecular engineering via living free radical polymerizations. *Macromol. Chem. Phys.,* 1998, vol. 199, 923-935
- *Chem. Review,* 2001, vol. 101 (12), 3787
- **CHARLEUX et al.** *Advances in Polymer Science,* 1999, vol. 142, 1-69
- **HANSEN.** Solubility parameter values. *Solubility parameter values*
- **ERIC GRULKE.** Polymer Handbook. 519-559
- **C.M.HANSEN.** The three dimensional solubility parameters. *J.Paint Technol.,* 1967, vol. 39, 105